(19) Europäisches Patentamt / European Patent Office / Office européen des brevets

(11) **EP 4 230 131 A1**

(12) **EUROPEAN PATENT APPLICATION**

(43) Date of publication:
**23.08.2023 Bulletin 2023/34**

(51) International Patent Classification (IPC):
**A61B 5/145** (2006.01)      **A61B 5/00** (2006.01)

(21) Application number: **22158049.1**

(22) Date of filing: **22.02.2022**

(52) Cooperative Patent Classification (CPC):
**A61B 5/14532; A61B 5/6826; A61B 5/6831;**
**A61B 5/6843; A61B 5/6844;** A61B 5/0059;
A61B 5/1172; A61B 5/742; A61B 2562/0233;
A61B 2562/0247; A61B 2562/029

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **DiaMonTech AG**
**10245 Berlin (DE)**

(72) Inventors:
• **LUBINSKI, Thorsten**
  **10245 Berlin (DE)**
• **SCHRIEK, Uwe**
  **10627 Berlin (DE)**

(74) Representative: **Lucke, Andreas**
**Boehmert & Boehmert**
**Anwaltspartnerschaft mbB**
**Pettenkoferstrasse 22**
**80336 München (DE)**

(54) **WEARABLE DEVICE AND METHOD FOR DETECTING AN ANALYTE IN TISSUE OF A HUMAN OR ANIMAL SUBJECT**

(57)      Disclosed herein is a wearable device (100) for detecting an analyte in tissue of a human or animal subject, the wearable device (100) comprising a holding portion (104) for securing the wearable device (100) to a body part (106), said holding portion (104) being configured to be arranged around the body part (106) of the subject so as to extend circumferentially around the body part (106) at least in part; wherein the wearable device (100) comprises a measurement body (16) and actuated means (110, 206, 208) for temporarily increasing a contact pressure between a measurement body (16).

Fig. 7a

EP 4 230 131 A1

## Description

FIELD OF THE INVENTION

The present invention generally relates to devices and methods for detecting an analyte in tissue of a human or animal subject. In particular, the present invention relates to devices and methods for non-invasive measurement of analytes in body fluids, such as glucose concentrations in human skin, in particular in the interstitial fluid of human skin.

BACKGROUND OF THE INVENTION

The present invention relates to a method of detecting an analyte in tissue of a human or animal subject. The method comprises a measurement procedure, in which the skin of the subject is brought in contact with a measurement body, which permits heat and/or pressure waves generated by absorption of excitation radiation in the tissue to be transferred to said measurement body. Excitation radiation is irradiated into the tissue to be absorbed by the analyte contained therein, wherein the intensity of said excitation radiation may be time-modulated, and wherein said excitation radiation may comprise radiation of different analyte-characteristic wavelengths that are irradiated one or both of simultaneously and sequentially.

Analyte-characteristic wavelengths as understood herein are wavelengths that allow for determining the presence of an analyte by wavelength-selective absorption, and hence form the basis of the analysis. As such, analyte-characteristic wavelengths may in particular include wavelengths corresponding to absorption maxima of the analyte. Further analyte-characteristic wavelengths are wavelengths corresponding to local minima between two absorption peaks. Namely, the difference between a local absorption minimum and an adjacent peak is a good measure of the concentration of analyte in the tissue. Herein, the term "local minimum" indicates that the absorptivity of the analyte at the given wavelength is smaller than at nearby wavelengths, but is still appreciable, otherwise they would not be characteristic for the analyte. In particular, in preferred embodiments, the absorptivity at these local minima serving as analyte-characteristic wavelengths is more than 5%, preferably more than 10%, more preferably more than 20%, most preferably more than 30% of the highest absorption peak associated with any of the analyte-characteristic wavelengths relied on in the analyte measurement. While wavelengths exactly corresponding with the absorption peaks or local absorption minima are the usually the preferred choices for the analyte-characteristic wavelengths, wavelengths close to the maxima/minima or between maxima and minima may also be used. Accordingly, as understood herein, "analyte-characteristic wavelengths" are also wavelengths where the difference in absorption to that at the closest absorption peak or the closest local absorption minimum is less than 30%, preferably less than 20% of the difference in absorption between the closest absorption peak and closest local absorption minimum. Analyte-characteristic wavelengths may also include wavelengths where the absorption of other substances with which the analyte is mixed in the tissue, is particularly low.

Further, a physical response of the measurement body, or of a component included therein, to heat and/or pressure waves received from said tissue upon absorption of said excitation radiation is detected using a detection device which generates a response signal based on said detected physical response, said response signal being indicative of the degree of absorption of excitation radiation. In the following, the steps of irradiating excitation radiation into the tissue to be absorbed by the analyte contained therein, detecting said physical response of the measurement body and generating said response signal may also be referred to as "performing an analyte measurement".

The present invention is not limited to any specific physical response to heat and/or pressure waves received from said tissue upon absorption of the excitation radiation, nor to any specific way of detecting this physical response in a manner that allows for generating a response signal that is indicative of the degree of absorption of excitation radiation. Various physical responses and corresponding detection methods have been previously proposed for these types of analyte measurement procedure by the present applicant and are briefly summarized below, and each of them may be applied in the present invention.

For example, the detection device may comprise a light source for generating a detection light beam travelling through at least a portion of said measurement body or a component included in said measurement body, and said physical response of the measurement body to heat and/or pressure waves received from said tissue upon absorption of said excitation radiation may be a local change in the refractive index of said measurement body or said component. In this case, the detection device may be configured for detecting one of a change in the light path or a change in the phase of detection light beam due to said change in refractive index of the material of the measurement body or the component included in the same.

For example, in various methods and devices described in detail in two earlier applications of the present applicant, published as WO 2015/193310 A1 and WO 2017/097824 A1, both of which included herein by reference, the measurement body is transparent for said detection light beam, and the detection light beam is directed to be totally or partially reflected at a surface of said measurement body that is in contact with said tissue. In this case, the detection device may comprise a photodetector, in particular a position sensitive photodetector which is capable of detecting a degree of deflection, in particular a deflection angle of said detection light beam due to said local change in refractive index. Accordingly, in this case, the physical response to the heat and/or pressure waves received by the measurement body is a local change in refractive index, and the response signal is the detected degree of deflection which is indeed found to be indicative of the degree of absorption of the excitation radiation.

In alternative variants suggested by the present applicant, as for example disclosed in international application PCT/EP2019/064356, included herein by reference, said detection device may comprise an interferometric device allowing for assessing said change in phase of the detection beam and generating a response signal indicative of said change in phase. In this case, the physical response of the measurement body (or a component included therein) to heat and/or pressure waves received from said tissue upon absorption of said excitation radiation is again a local change in index of refraction, while the response signal is in this case an interferometric signal reflecting a change in the phase of the detection beam due to the local change in refractive index.

In yet alternative embodiments, the measurement body or a component in said measurement body may have electrical properties that change in response to a local change in temperature and/or a change in pressure associated therewith, and said detection device comprises electrodes for capturing electrical signals representing said electrical properties. Various possible setups are disclosed in WO 2019/110597 A2, included herein by reference. For example, the measurement body may comprise sections having piezoelectric properties, and pressure changes associated with received heat and/or pressure lead to electrical signals that can be recorded with the electrodes. In this case, the change in pressure resembles the physical response of the measurement body or of a component included therein, to heat and/or pressure received from the tissue upon absorption of the excitation radiation, which is detected using the piezoelectric properties of the measurement body and the electrode, and which leads to electrical signals representing the aforementioned response signal that is indicative of the degree of absorption of the excitation radiation. In yet further variants, a temperature change due to received heat can be directly measured using very sensitive temperature sensors.

In some examples, the measurement body or a component in said measurement body may comprise a volume in which acoustic waves such as sound waves can propagate, for example a cavity or void, a recess or cut-out and/or an acoustic resonator. The physical response of the measurement body or of said component in the measurement body may thus be a sound wave, e.g. a longitudinal pressure wave, in said volume that is excited by a heat and/or pressure wave received from the tissue upon absorption of the excitation radiation. The detection device may comprise a microphone that is arranged in or in contact with said volume. The microphone may act as a transducer to convert sound waves into electrical signals, wherein the electrical signals may represent the aforementioned response signal that is indicative of the degree of absorption of excitation radiation.

Note that in the following description, the physical response of the measurement body to heat received from the tissue as a result of thermal contact between the measurement body and the tissue is described in detail. However, it is to be understood that in various embodiments of the device and method of the invention, the tissue is in pressure transmitting contact with the measurement body, and the physical response of the measurement body is a response to pressure waves received from the tissue. Herein, the expression "pressure transmitting contact" shall include all relations that allow for the transfer of pressure waves from the tissue to the measurement body, and in particular, an acoustically coupled relation, where the coupling could be established by a gas, a liquid or a solid body. All detailed explanations given in connection with the thermal contact and physical response to heat received by the measurement body from the tissue shall be understood in combination with scenarios including pressure transmitting contact and physical response to pressure waves, where applicable, without explicit mention.

Note that the term "analyte measurement procedure" or "analyte measurement" indicates that this measurement procedure is based on response signals obtained with excitation radiation at analyte-characteristic wavelengths.

The method may further comprise an analyzing step, in which said analyzing is carried out based, at least in part, on said response signal. Since in this case, the response signal is indicative of the degree of absorption of excitation radiation comprising "analyte-characteristic wavelengths", the response signal is directly related to the concentration of the analyte in the tissue. Accordingly, the analyzing step is based at least in part on a measure of the concentration of the analyte in the tissue, and in some non-limiting applications, it may actually amount to determining this concentration.

For example, the above method has been employed by the applicant in devices for non-invasive measuring of a glucose level of a user. In this specific application, the "analyte" is formed by glucose, and the "tissue" is the skin of the user. It has been previously demonstrated that this method allows for very precise measurement of glucose concentration in the interstitial fluid within the skin of a person, which is found to be directly related with and hence representative of the glucose content of the patient's blood. Shown in Fig. 4 of the present application is the result of a Clark's error grid analysis taken from WO 2017/097824 A1, demonstrating that the above analysis method allows for predicting the actual glucose concentration of a person very precisely.

Recent advances in the miniaturization of components such as lasers for generating excitation radiation at a plurality of analyte-characteristic wavelengths enable the implementation of a method for detecting an analyte as described above in ever small devices. The method could for example even be implemented in wearable devices that can be worn on the human body such as smartwatches, fitness trackers or chest-strap monitors (e.g. heart-rate monitors). For a reliable detection of the analyte, however, close contact has to be established and maintained between the measurement body and the skin of the subject. This is challenging for wearable devices like smartwatches, which typically are worn loosely on the body.

SUMMARY OF THE INVENTION

It is thus an object of the present invention to provide means that allow for a reliable and accurate detection of an analyte in tissue of a human or animal subject by a method as set forth above that is implemented in a wearable device.

This object is met by a wearable device for detecting an analyte in tissue of a human or animal subject according to claim 1, a method for detecting an analyte in tissue of a human or animal subject using a wearable device according to claim 12 and a wearable device for detecting an analyte in tissue of a human or animal subject according to claim 13. Examples of the present invention are detailed in the dependent claims.

According to a first aspects of the invention, a wearable device for detecting an analyte in tissue of a human or animal subject according to the invention comprises a measurement body having a contact surface suitable to be brought in contact with a skin of the subject, said contact permitting heat and/or pressure waves generated by absorption of excitation radiation in the tissue to be transferred to said measurement body. The wearable device further comprises an excitation radiation source configured for irradiating excitation radiation at a plurality of wavelengths into the tissue to be absorbed by the analyte contained therein. The wearable device also comprises a detection device for detecting a physical response of the measurement body or of a component included therein to a heat and/or pressure wave received from the tissue upon absorption of the excitation radiation and for generating a response signal based on said detected physical response, said response signal being indicative of a degree of absorption of excitation radiation. The wearable device further comprises a controller to control the excitation radiation source to irradiate excitation radiation into the tissue to be absorbed by the analyte contained therein and to control the detection device to detect said physical response and to generate a response signal indicative of the degree of absorption of said excitation radiation. The wearable device also comprises a holding portion for securing the wearable device to a body part, in particular a limb of the subject, said holding portion being configured to be arranged around the body part of the subject so as to extend circumferentially around the body part at least in part. The wearable device further comprises actuated means for temporarily increasing a contact pressure between the measurement body and the skin of the subject by reversibly performing one or more of the following: (1) reducing an inner diameter of the wearable device, wherein the inner diameter of the wearable device is a distance between two opposing portions of the wearable device being arranged on opposite sides of the body part when the wearable device is secured to the body part; (2) reducing an inner circumference of the wearable device, wherein the inner circumference of the wearable device is a length of an inner surface of the wearable device facing the body part when the wearable device is secured to the body part; and (3) moving the measurement body or a part thereof radially inward towards the body part.

The wearable device is configured to be worn by the subject by securing or fastening the wearable device to said body part using the holding portion. In other words, physical dimensions and the weight of the wearable device are such that the wearable device can be carried by the subject, e.g. such that the wearable device can be worn by the subject over extended periods of time (e.g. for more than one hour) or permanently. In some embodiments, the wearable device can for example be a smartwatch, a fitness tracker or a chest-strap monitor. The wearable device may be configured for use on human subjects (e.g. a user or a patient), but may also be adapted for use on animal subjects, e.g. by adjusting the physical dimensions and/or the shape of the wearable device accordingly, for example so as to fit on the respective body part. The animal subjects may be mammals, in particular larger mammals (e.g. mammals with a body size on the same order of magnitude as humans) such as cats, dogs, pigs and cows.

The analyte is not particularly limited and may be any substance that absorbs excitation radiation at a plurality of analyte-characteristic wavelengths. Preferably, the analyte is a physiologically relevant substance that is present in tissue of humans and/or animals. The analyte may in particular be glucose.

The tissue may be any tissue in the human or animal body that can be irradiated by excitation radiation and from which heat and/or pressure waves may be transferred to the measurement body. The tissue may for example be sufficiently close to the skin of the subject for the tissue to be reached by excitation radiation irradiated through the skin of the subject and/or for heat and/or pressure waves generated by absorption of the excitation radiation in the tissue to propagate to the surface of the skin. The tissue may in particular be the skin of the subject. In one example, the analyte is glucose, in particular glucose present in an interstitial fluid of the skin, i.e. in a fluid present in a space between cells within the skin. While in the following explanations and in the description of the specific embodiments reference may be made to this embodiment, it is to be understood that the invention is not limited to this, and that it can be applied to other types of tissue and/or analytes.

The measurement body may be any body that (or a component thereof) exhibits a physical response to heat and/or pressure waves that can be detected with a detection device, e.g. as described above. The measurement body may be a solid body, in particular a transparent solid body. In some embodiments, the body may also comprise a fluid, which may e.g. be contained in a cavity or void within the measurement body. The contact surface may be a surface of the measurement body that is exposed from the wearable device, i.e. not covered by any other part of the wearable device, such that it can be brought in contact with the skin of the subject. The contact surface may be a simply connected surface (i.e. without holes), but may also for example be a surface extending around a recess or cut-out in the measurement body (e.g. an edge or rim of the recess). The contact between the measurement body and the skin may be a thermal contact that permits the transfer of heat waves to the measurement body, e.g. through the contact surface, and/or may be a pressure transmitting contact that permits the transfer of pressure waves to the measurement body, e.g. through the contact surface and/or through an interface or plane surrounded by the contact surface (e.g. a "surface" or opening of a recess in the measurement body that is surrounded by the contact surface).

The excitation radiation source is configured to generate excitation radiation at a plurality of wavelengths ("excitation wavelengths"), in particular at a plurality of analyte-characteristic wavelengths. Said plurality of wavelengths or analyte-characteristic wavelengths may for example comprise between 2 and 50 wavelengths, in some examples between 5 and 20 wavelengths. The excitation radiation source may for example be a light source such as a laser that is configured to generate excitation radiation in the optical spectrum, for example in the infrared spectrum, in particular in the near-infrared and/or mid-infrared spectrum. For irradiating the excitation radiation generated by the excitation radiation source into the tissue, e.g. through the contact surface and/or through the measurement body, the wearable device may comprise one or more optical elements such as waveguides, lenses and/or mirrors.

In some embodiments, said excitation radiation is generated using an array of lasers, in particular semiconductor lasers, further in particular quantum cascade lasers, each having a dedicated wavelength. Each of the lasers may have its own modulation device, or they may have a common modulation device and they may be controlled by a common or by individual control units. The lasers of an array may be optically aligned in a way that allows for using a common optical path for the excitation beam and they may for example radiate into the tissue through a common light waveguide. The laser array may be combined on a single semiconductor chip.

In some embodiments, said excitation radiation is generated using at least one tunable laser, in particular at least one tunable quantum cascade laser.

In a preferred embodiment, some or all of said excitation wavelengths are in a range of 5 $\mu$m to 13 $\mu$m, preferably 8 $\mu$m to 11 $\mu$m. In alternative embodiments, some or all of said excitation wavelengths are in a range of 3 $\mu$m to 5 $\mu$m. This wavelength range is for example useful for detecting absorption of $CH2$ and $CH_3$ vibrations in fatty acids.

The detection device may be any device that is configured to detect the physical response of the measurement body or of the component therein to heat and/or pressure waves and to generate a corresponding response signal, e.g. as detailed above. The detection device may comprise one or more detection elements such as a photodetector, an electrode, a temperature sensor and/or a microphone for detecting the physical response. The detection device may further comprise one or more probing sources such as a light source and/or a current or voltage source for generating a probing excitation such as a light beam and a current or voltage, respectively, that is to be detected by the one or more detection elements, e.g. to detect an effect of a heat and/or pressure wave on the probing excitation such as a deflection of the light beam. The response signal generated by the detection device may allow for quantifying the physical response of the measurement body and thereby the degree of absorption of excitation radiation in the tissue.

In some embodiments, said detection device comprises a light source for generating a detection light beam travelling through at least a portion of said measurement body or a component included in said measurement body, said physical response of the measurement body to heat received from said tissue upon absorption of said excitation radiation is a local change in the refractive index of said measurement body or said component, and said detection device is configured for detecting one of a change in the light path or a change in the phase of detection beam due to said change in refractive index and for generating a response signal indicative of said change in light path or phase of the detection beam.

In some embodiments, said measurement body is transparent for said detection light beam, said detection light beam is directed to be totally or partially reflected at a surface of said measurement body that is in contact with said skin, and wherein said detection device comprises a photodetector, in particular a position sensitive photodetector, capable of detecting a degree, in particular angle of deflection of said detection light beam due to said local change in refractive index.

In some embodiments, said detection device comprises an interferometric device allowing for assessing said change in phase of the detection beam and generating a response signal indicative of said change in phase.

In some embodiments, said measurement body or a component in said measurement body has electrical properties that change in response to a local change in temperature or a change in pressure associated therewith, and wherein said detection device comprises electrodes for capturing electrical signals representing said electrical properties.

The controller is configured to perform analyte measurements for detecting the analyte in said tissue, i.e. to control the excitation radiation source to irradiate excitation radiation, in particular excitation radiation at a plurality of analyte-characteristic wavelengths, into the tissue to be absorbed by the analyte contained therein and to control the detection device to detect said physical response and to generate a response signal indicative of the degree of absorption of said excitation radiation. The controller may be configured to control the excitation radiation source such that the intensity of said excitation radiation is time-modulated (e.g. pulsed) and/or such that different analyte-characteristic wavelengths that are irradiated one or both of simultaneously and sequentially.

The controller may be embodied in hardware, software or both. In particular, the controller may comprise one or more computers, processors, microcontrollers, FPGAs ASICs and corresponding computer programs which when executed on the corresponding hardware provide the control functions described herein. In some examples, the controller may be a distributed system, for example comprising several control units that are in data communication with each other, of which some may be provided in a housing of the wearable device, and others remote from the housing, but it can also be formed by a single control unit. To control components of the wearable device such as the excitation radiation source and the detection device, the controller may be configured to generate digital and/or analog control signals for the respective component. In a preferred embodiment of the wearable device, said controller is configured to control components of the wearable device for carrying out a method for detecting an analyte in tissue of a human or animal subject according to any one of the embodiments described herein at least in part, in one example in its entirety (i.e. to execute all steps of said method).

The controller may further be configured to perform an analyzing step, for example as described above. The controller may in particular be configured to determine a measure of the concentration of the analyte in the tissue, e.g. the concentration itself or a quantity that has a known functional relation to the concentration (e.g. is proportional to the concentration), based on the response signal.

When the excitation radiation is radiated into the tissue, part of it will be absorbed along its light path, e.g. at different depths below the surface (i.e. the skin), for example by the analyte contained within the tissue. In response to the absorption, a heat signal is generated, which diffuses back to the surface/skin and is received by the measurement body. When the intensity of the excitation radiation is modulated, the tissue is heated by absorption in a time-dependent manner, leading to a temperature field that varies as a function of space and time and that is often referred to as a thermal wave. The term thermal "wave" is somewhat misleading, since the travel of heat through the material is not governed by a wave equation, but by a diffusion equation instead. This also means that the largest depth of the modulated absorption underneath the surface that can be detected by heat received by the measurement body at the surface is limited approximately by the so-called diffusion length $\mu_t$, which depends on the density p, the specific heat capacity $C_p$, and the thermal conductivity $k_t$ of the tissue as well as on the modulation frequency f of the excitation radiation:

$$\mu t(f) = \sqrt{\frac{k_t}{\rho \cdot C_p \cdot 2\,f}}$$

Since the interstitial fluid is only found in the tissue at a certain range below the surface of the skin, in order to generate response signals indicative of absorption in the interstitial fluid, the modulation frequency of the excitation radiation has to be indeed "sufficiently low" such that the diffusion length $\mu_t$ is long enough to cover the distance between regions with interstitial fluid and the skin surface. However, as explained above, the diffusion length depends on the physical properties of the tissue, namely density p, the specific heat capacity $C_p$, and the thermal conductivity $k_t$, and the present applicant noticed that these properties will not only vary from person to person, but may also vary for the same person with time, depending for example on whether the skin is dry or moist, whether the skilled person has used hand moisture lotion, or the like. In other words, the thermal diffusion length as a function of frequency is a material/tissue status that for best results should be assessed at or close to the time of the analyte measurement procedure in a material/tissue status analyzing procedure, e.g. as described in WO 2021/233559 A1, included herein by reference. Thereby, a modulation frequency may be determined that is "sufficiently low" such that the response signal reflects at least in part absorption of excitation radiation within the interstitial fluid.

Moreover, the depth below the surface at which the interstitial fluid resides is found to be dependent on the thickness of the stratum corneum, which again does not only vary from person to person, but also varies for the same person over time and also over the location of the skin surface where the measurement takes place. For example, if a person has recently done physical work with his or her hands, or has practiced a string instrument, the stratum corneum may be thicker than usual, meaning that the absorption has to be effected in deeper layers below the surface of the skin in order to cover the interstitial fluid. Again, the thickness of the stratum corneum is a "material/tissue status" that can be assessed in said material/tissue status analyzing procedure.

However, even if the thermal diffusion length is sufficiently long that the response signal reflects at least in part absorption of excitation radiation within the interstitial fluid, the response signal will also represent any absorption in upper layers of the skin, in particular the stratum corneum, and hence include a contribution that is not related to the glucose within the interstitial fluid. Indeed, this contribution from upper layers of the skin, in particular the stratum corneum, will be typically overrepresented in the response signal, since the intensity of the excitation radiation in the upper layers is higher than deeper into the skin, and since the absorption heat needs to travel only a shorter distance from the upper layers to reach the measurement body. In order to estimate this contribution of higher layers of the skin to the (first) response signal, at least one additional second response signal may be recorded in response to excitation radiation with a second modulation frequency, which is higher than the other (first) modulation frequency, and hence leads to a shorter diffusion length and therefore represents absorption predominantly in the upper layers of the skin above the region where the interstitial fluid resides. Then, response signals corresponding to said modulation frequencies, or quantities derived therefrom, are mathematically combined to yield information more specifically indicative of the absorption in the interstitial fluid. There are different ways of mathematical combining these response signals, and this aspect of the invention is not limited to any single one of them. For example, the second response signal can be multiplied with a normalizing factor that is obtained e.g. based on reference measurements with excitation wavelength(s) for which glucose absorption is negligible, and then the product may be subtracted from the first response signal.

The holding portion is configured to secure the wearable device to a body part of the subject such as a limb. Preferably, said body part is an upper limb of the subject such as an arm or a part thereof, in particular an upper arm and/or a wrist. The holding portion is configured to be arranged around said body part such that the holding portion extends around the body part along a circumferential direction (e.g. substantially parallel to a surface of said body part). In other words, when arranged around said body part, the holding portion extends around a circumference of the body part. When arranged around said body part, the wearable device and/or the holding portion may extend around the body part completely (by 360°, e.g. to form a closed loop enclosing said body part such as a closed wristband) or partially (by less than 360°, e.g. to form a bracket- or clip-like structure such as an open wristband). The wearable device and/or the holding portion may for example extend around the body part by between 180° and 360°, preferably between 270° and 360°. In some examples, the holding portion may have a substantially circular or substantially elliptical shape. In some embodiments, the holding portion or a part thereof may be attached to a main housing of the wearable device, wherein the main housing may e.g. contain or hold some or all of the measurement body, the excitation radiation source, the detection device and the controller. A first end of the holding portion may for example be attached to a first side of the main housing and a second end of the holding portion maybe attached to a second side of the main housing opposite to the first side, e.g. so as to form a closed loop. The main housing may be part of the holding portion, e.g. to close the loop, and in some examples may be formed integrally with the holding portion or a part thereof.

The wearable device is configured to temporarily increase the contact pressure between the measurement body and the skin of the subject, e.g. to establish and maintain close contact for performing an analyte measurement. The wearable device may for example be configured to temporarily increase the contact pressure to between 0.1 N/cm² and 100 N/cm², preferably to between 1 N/cm² and 10 N/cm². The wearable device is further configured to reduce or release said temporarily increased contact pressure, e.g. after completion of the measurement.

For this, the wearable device comprises actuated means that are configured to reduce and/or increase the inner diameter of the wearable device (e.g. an inner diameter of the holding portion arranged around said body part) and/or the inner circumference of the wearable device (e.g. a length of an inner surface of the holding portion from a first end of said holding portion along the circumferential direction around said body part to a second end of said holding portion opposite to said first end). Additionally or alternatively, said actuated means may also be configured to move the measurement body or a part thereof, in particular the contact surface, radially inward towards said body part and/or radially outward away from said body part. Loosely speaking, the actuated means are configured to tighten the wearable device (e.g. the holding portion) and/or to move the measurement body closer to the body part in order to ensure a tight fit and close contact between the measurement body and the skin. Preferably, the controller is configured to control the actuated means to temporarily increase the contact pressure.

In the context of this disclosure, "radially" or a "radial direction" may refer to a direction that is perpendicular or substantially perpendicular (e.g. at an angle between 70° and 110°) to the surface (e.g. the skin) of said body part (and thus perpendicular or substantially perpendicular to the circumferential direction extending around the body part). A first element that is closer to the body part than a second element (i.e. displaced radially inward from the second element) maybe referred to as an "inner element", whereas the second element maybe referred to as an "outer element".

Said actuated means for temporarily increasing the contact pressure may for example comprise one or more actuators, wherein the one or more actuators may for example include one or more of an electric actuator (e.g. an actuator comprising an electric motor), a magnetic actuator (e.g. an actuator configured to generate or switch a magnetic field to move a magnetic object or an electromagnet), a piezoelement (e.g. an element comprising or consisting of a piezoelectric material) and a fluid pump.

Additionally or alternatively, said actuated means for temporarily increasing the contact pressure may comprise one or more deformable members configured to be deformed, extended and/or retracted reversibly. The one or more deformable members may for example comprise one or more of a bimetallic element (e.g. an element such as a strip or coil comprising two or more materials, in particular metals, having different coefficients of thermal expansion), a shape-memory alloy structure (e.g. a structure or element that has a first shape (e.g. straight) at a first temperature and a second shape (e.g. bent) at a second temperature different from the first temperature), a bistable spring assembly (e.g. an assembly comprising one or more springs that can be compressed/stretched between two stable configurations, which may for example be defined by the arrangement of the springs and/or respective mechanical stops or detents), a pneumatically and/or hydraulically extendable member (e.g. a hydraulic or pneumatic cylinder or a pneumatically or hydraulically movable shaft or piston) and an inflatable body (e.g. a body enclosing a volume that is configured to receive a fluid such as air to be expanded from a compressed state to an expanded state).

In one example, the actuated means comprises a fluid pump and one or more inflatable bodies. The one or more inflatable bodies may for example be arranged along the holding portion in the circumferential direction, e.g. such that the inflatable bodies are arranged circumferentially around the body part when the wearable device is secured to the body part. The fluid pump may be configured to inflate the inflatable bodies, e.g. by pumping ambient air into the inflatable bodies, to increase a radial extent of the inflatable bodies and thus of the holding portion, thereby reducing the inner circumference of the wearable device. In other words, the actuated means may be configured to inflate the holding portion in order to temporarily increase the contact pressure.

By providing actuated means for temporarily increasing a contact pressure between the measurement body and the skin of the subject, the contact pressure can be increased automatically, e.g. by the controller, prior to performing an analyte measurement. Thereby, one can ensure that close contact between the measurement body and the skin is established and maintained throughout the measurement. This allows for an accurate and reliable detection of analytes in tissue of human or animal subjects with wearable devices such as smartwatches. After performing the analyte measurement, the temporarily increased contact pressure may be reduced or released again (e.g. by increasing the inner diameter and/or the inner circumference of the wearable device and/or by moving the measurement body radially outward away from the body part) as maintaining an increased pressure for extended periods of time may be uncomfortable for the subject.

At least one of the one or more actuators and/or at least one of the one or more deformable members may be arranged on a same side or an opposite side of the body part as the measurement body when the wearable device is secured to the body part. The respective actuator and/or deformable member may for example be arranged adjacent to the measurement body, e.g. next to the measurement body or behind the measurement body as seen from the body part, or in a portion of the wearable device that opposes the measurement body (i.e. is rotated by about 180° around the body part with respect to the measurement body). Compared to a lateral arrangement of the respective actuator and/or deformable member, this may be advantageous for achieving a larger increase in the contact pressure for a given reduction of the inner diameter and/or the inner circumference of the wearable device.

In some embodiments, the measurement body is mounted movably on and/or in the wearable device. The one or more actuators may comprise an actuator configured to move the measurement body radially inward towards the body part, e.g. a piezoelement on which the measurement body is mounted or an actuator configured to move a shaft or piston on which the measurement body is mounted. Additionally or alternatively, the one or more deformable members may comprise a deformable member configured to move the measurement body radially inward towards the body part, e.g. an inflatable body such as a fluid-filled cushion on which the measurement body is mounted or a pneumatically and/or hydraulically extendable member on which the measurement body is mounted.

In some embodiments, the holding portion comprises a plurality of segments (e.g. between 2 and 20 segments, in some examples between 4 and 10 segments) that are connected with each other to form a chain (i.e. a linear sequence of segments). The segment may be connected with each other so as to allow a relative movement, in particular tilting or rotation, of the segments with respect to each other, e.g. to provide a certain flexibility in the holding portion. The holding portion may for example comprise a plurality of coupling member such as hinges, each of which may be arranged between a respective pair of adjacent segments. In some examples, some or all of the segments may have an identical shape.

The actuated means for temporarily increasing the contact pressure may be configured to reversibly reduce a circumferential extent of one or more segments of the holding portion, e.g. a length of the respective segment along the circumferential direction around the body part, and/or a distance between one or more pairs of adjacent segments of the holding portion. The actuated means may for example comprise a deformable member such as an inflatable body or an extendable member that is configured to adjust the circumferential extent of a segment (e.g. by deflating or contracting the segment) and/or to adjust the circumferential extent of a coupling member (e.g. by deflating or contracting the coupling member).

The actuated means for temporarily increasing the contact pressure may comprise at least one actuated hinge. The actuated hinge may connect a pair of adjacent segments of the holding portion. The actuated hinge may be configured to rotate the pair of adjacent segments with respect to each other, e.g. to move the actuated hinge radially inward towards the body part. The actuated hinge may for example comprise or consist of a shape-memory alloy or a bimetallic element (e.g. a bimetallic coil) and/or may comprise an actuator such as a piezoelement or an electric actuator or a pneumatically and/or hydraulically extendable member.

The plurality of segments may comprise a first segment in and/or on which the controller is arranged. The first segment may for example be a main housing of the wearable device, which may also contain some or all of the other components of the wearable device apart from the holding portion. The main housing may further comprise a display, wherein the display may e.g. be configured to display a measurement result that is indicative of the degree of absorption of the excitation radiation and/or of a presence and/or a concentration of the analyte in the tissue. The display may for example be configured to display the concentration of the analyte determined by the controller.

In some embodiments, the excitation radiation source may be arranged in or on a different segment of the holding portion than the controller and/or the display. In other words, the plurality of segments may comprise a second segment in and/or on which the excitation radiation source is arranged, the second segment being different from the first segment. This may for example be advantageous since the excitation radiation source may generate a sizeable amount of heat when irradiating excitation radiation into the tissue. The controller and/or the display, which may e.g. be an organic light-emitting diode (OLED) display, may be sensitive with regard to temperature changes. In one example, the second segment is adjacent to the first segment.

The second segment may be larger than the first segment in at least one physical dimension. For example, a circumferential extent and/or a volume of the second segment may be larger than a circumferential extent and a volume, respectively, of the first segment. This may for example allow for dissipating a larger amount of heat from the second segment. In one example, the second segment is larger than any other segment of the plurality of segments segment in at least one physical dimension, e.g. in the circumferential extend and/or in the volume.

In some embodiments, the measurement body and the excitation radiation source may be arranged in different parts of the wearable device, in particular in different segments of the holding portion. In other words, the measurement body and the excitation radiation source may be positioned spaced apart from each other, e.g. such that the measurement body is rotated by an angle between 30° and 330° (e.g. between 30° and 60°), in some examples between 90° and 270°, in one example between 150° and 210°, along the circumferential direction with respect to the excitation radiation source. This may for example allow for arranging the measurement body such that, when the wearable device is secured to a wrist or lower arm of a human subject, the measurement body is arranged on an inside of the wrist or lower arm (i.e. facing towards the torso of the subject) when the main housing (e.g. the first segment) of the wearable device is arranged on an outside of the wrist or lower arm (i.e. facing away from the torso). In other words, the main housing (which may e.g. house the display) and the measurement body may be arranged on opposite sides of the wearable device. The excitation radiation source may for example be arranged in or on the main housing or in or on a segment adjacent to the main housing. In another example, the measurement body is arranged in or on the main housing and/or the first segment and the excitation radiation source is arranged in or on the second segment, which may e.g. be adjacent to the first segment.

The wearable device may further comprise a waveguide configured to guide excitation radiation from the part of the wearable device in which the excitation radiation source is arranged to the part of the wearable device in which the measurement body is arranged. The waveguide may for example be an optical fiber, e.g. a multimode optical fiber. The waveguide may extend along a part of the holding portion, e.g. through one or more segments and/or one or more coupling members. In one example, the waveguide extends from the excitation radiation source to the measurement body, e.g. to guide excitation radiation from an output of the excitation radiation source to the measurement body and to couple the excitation radiation into the measurement body.

In a preferred embodiment, the wearable device further comprises a spacer for thermally insulating the body part from the excitation radiation source. The spacer may be arranged between the excitation radiation source and an inner surface of the wearable device facing the body part when the wearable device is secured to the body part. A surface of the spacer may in particular form a part of the inner surface of the wearable device. The spacer may for example comprise or consist of a material having a lower thermal conductivity than a material that the holding portion (e.g. the segments of the holding portion and/or a frame or housing of the holding portion or of the segments) and/or the main housing of the wearable device is/are made of. In some examples, the thermal conductivity of the spacer material may be at least a factor of two, preferably at least a factor of five, in one example at least a factor of ten smaller. Additionally or alternatively, the spacer may be configured to maintain at least a minimum distance between the skin of the subject and the excitation radiation source and/or the inner sidewall of the second segment. The spacer may for example comprise a plurality of ridges or fins that are configured to prevent the inner sidewall of the second segment from coming in contact with the skin.

In some embodiments, the measurement body or a part thereof permanently protrudes from the inner surface of the wearable device. Additionally or alternatively, the measurement body may be arranged in and/or on a permanent protrusion on the inner surface of the wearable device. The contact surface of the measurement body may for example protrude by a distance between 0.2 mm to 5 mm, in some examples between 0.5 mm and 2 mm from an adjacent portion of the inner surface of the wearable device, e.g. from a portion of the inner surface surrounding the measurement body. This may further increase the contact pressure that can be achieved between the measurement body and the skin.

The wearable device may comprise a removable battery. The removable battery may be configured to be removably coupled to an integrated battery of the wearable device and/or to the excitation radiation source for supplying energy to the integrated battery and/or to the excitation radiation source. This may for example allow for recharging the integrated battery, e.g. prior to or after performing an analyte measurement. It may further allow for supplying energy to the excitation radiation source while irradiating excitation radiation into the tissue, at which time the energy consumption of the wearable device may be greatly increased. Preferably, the removable battery is configured such that it be coupled to the integrated battery and/or to the excitation radiation source without use of any tools and/or without having to open the wearable device (e.g. without opening the main housing or any other part of the wearable device and without removing any cover, cap or sealing). The removable battery may for example be configured to be clipped to or onto the wearable device by mechanical means (e.g. clips and/or hooks) and/or to be attached to the wearable device magnetically (e.g. by one or more magnets in each of the removable battery and the wearable device, for example the main housing or the holding portion). The removable battery may be configured to supply energy by a wired electrical connection (e.g. via a suitable connector or connecting pins) and/or a wireless electrical connection (e.g. inductive coupling or charging).

The wearable device may comprise an integrated battery, which may for example be mounted in the wearable device (e.g. in the main housing) permanently, e.g. such that it cannot be removed at all or only using tools such as a screwdriver and/or by opening the main housing. The integrated battery may for example be configured to supply energy to one or more of the detection device, the controller, the excitation radiation source, the actuated means and the display.

Energy consumption of the excitation radiation source for generating the excitation radiation may be too large to be supplied by the integrated battery and/or may quickly drain the integrated battery. The removable battery may be configured to be removably attached to the wearable device for supplying energy to the excitation radiation source while irradiating excitation radiation into the tissue. The removable battery may e.g. be attached to the wearable device whenever an analyte measurement is to be performed. In some examples, the integrated battery may not even be configured to supply energy to the excitation radiation source, i.e. may not be connected to the excitation radiation source.

The wearable device may further comprise one or more auxiliary sensors configured to generate a sensor signal that depends on the contact pressure between the measurement body and the skin of the subject. As used herein, an auxiliary sensor may for example be a sensor that is per se unrelated to the measurement apparatus for measuring the analyte absorption. In other words, an auxiliary sensor may be a sensor that is (primarily) used for other purposes than for detecting or monitoring the analyte. The auxiliary sensor may for example be a pressure sensor that is configured to measure the contact pressure directly, e.g. a capacitive pressure sensor or a piezoresistive strain gauge. Preferably, however the auxiliary sensor is a sensor that is primarily used for other purposes, but nonetheless allows for determining the contact pressure due to the sensor signal depending on the contact pressure (e.g. changing when the contact pressure varies). The auxiliary sensor may in particular be configured to measure one or more of a blood pressure of the subject, a pulse (or heart rate) of the subject and an oxygen saturation of blood of the subject. The auxiliary sensor may for example be an optical pulse and/or oxygen saturation sensor. The sensor signal may for example contain contributions originating from a flow of blood through the body part that the wearable device is secured to (e.g. a pulse time trace). When the contact pressure increases, said contributions may become larger and vice-versa (i.e. the "quality" of the sensor signal for e.g. the pulse measurement may improve). The contact pressure may for example be determined (estimated) from a peak-to-peak amplitude of the sensor signal and/or based on a Fourier analysis, e.g. based on a weight of a Fourier component associated with the subject's heart rate. Other examples of auxiliary sensors are a hydration sensor, a body temperature sensor, a breath rate sensor, a lactate sensor, a blood alcohol sensor, a carbon monoxide sensor, an urea sensor, a creatinine sensor, an albumin sensor, a bilirubin sensor and a hemoglobin sensor. The aforementioned sensors may for example be implemented as optical sensors working in the visible spectrum and/or in the infrared spectrum. Measurements of the one or more auxiliary sensors may be combined with analyte measurements (e.g. glucose measurements) and/or may contribute to an accurate detection of the analyte (e.g. a determination of the glucose level). In some embodiments, said one or more auxiliary sensors may be provided as part of a contact monitor as detailed below.

The controller may be configured to determine the contact pressure between the measurement body and the skin of the subject based on a response signal from the detection device and/or based on a sensor signal from the auxiliary sensor. Additionally or alternatively, the wearable device may comprise a contact monitor that is configured to determine the contact pressure between the measurement body and the skin of the subject, e.g. as detailed below. Said contact monitor may for example be configured to determine the contact pressure based on a response signal from the detection device and/or based on a sensor signal from the auxiliary sensor as described for the controller in the following. Determining the contact pressure may comprise determining the contact pressure quantitatively (i.e. determining a value of the contact pressure) and/or determining whether the contact pressure exceeds a threshold, for example a pre-defined threshold (e.g. whether the contact pressure is sufficiently high to reliably perform an analyte measurement).

The controller may for example be configured to determine the contact pressure by controlling the excitation radiation source to irradiate excitation radiation into the tissue to be absorbed therein at one or more wavelengths at which the degree of absorption of said excitation radiation in said tissue is substantially independent of a concentration of the analyte in the tissue and by controlling the detection device to detect said physical response and to generate a response signal indicative of the degree of absorption of said excitation radiation. In other words, the controller may determine the contact pressure based on a response signal obtained from a measurement performed at wavelengths at which the analyte does not absorb radiation or at which the absorption by the analyte is negligible compared to other absorption processes. The physical response of the measurement body or of the component included therein and thus the response signal may therefore also be substantially independent of a concentration of the analyte in the tissue. This may allow for determining the contact pressure without the measurement being influenced by the (unknown) concentration of the analyte.

The degree of absorption of said excitation radiation may for example be considered to be substantially independent of the concentration of the analyte if the absorptivity of the analyte in said tissue is less than 5%, preferably less than 2%, in some examples less than 1%, in one example less than 0.5% of the highest absorptivity at any of the analyte-characteristic wavelengths relied on in the analyte measurement and/or less than 30%, preferably less than 20%, in some examples less than 10%, in one example less than 5% of the lowest absorptivity at any of the analyte-characteristic wavelengths relied on in the analyte measurement. The one or more wavelengths used for determining the contact pressure may in particular be analyte-characteristic wavelengths of another substance that is different from the analyte, in particular a substance that is present in said tissue at a high and/or substantially constant concentration. Said substance may for example be water. The contact pressure may for example be determined based on a comparison of a value of said concentration determined based on the response signal with a known value of said concentration in said tissue and/or in the human/animal body.

The controller may be configured to discard a response signal generated by the detection device for detection of the analyte if the determined contact pressure is below a threshold, e.g. a pre-defined threshold. This may indicate that contact between the measurement body and the skin of the subject is not sufficiently close or tight to ensure a reliable transfer of heat and/or pressure waves generated by absorption of excitation radiation in the tissue. The controller may be configured to control the actuated means to further increase the contact pressure as described above in response to determining that the determined contact pressure is below the threshold. Additionally or alternatively, the controller may also be configured to repeat the analyte measurement at a later point in time, e.g. after a delay time.

In some embodiments, the wearable device may further comprise a contact monitor for monitoring a thermal contact state and/or a pressure transmitting contact state between the contact surface of the measurement body and the skin of the subject, e.g. as detailed below for the wearable device according to the second aspect of the invention.

The present invention further provides a method for detecting an analyte in tissue of a human or animal subject using a wearable device according to any one of the embodiments described herein. The method comprises securing the wearable device to a body part, in particular a limb of the subject such that the contact surface of the measurement body faces a skin of the subject. A contact pressure between the measurement body and the skin of the subject is increased temporarily by one or more of reducing the inner diameter of the wearable device, reducing the inner circumference of the wearable device and moving the measurement body radially inward towards the body part. While maintaining the temporarily increased contact pressure, excitation radiation is irradiated into the tissue to be absorbed therein using the excitation radiation source. Using the detection device, the physical response of the measurement body or a component included therein is detected and a response signal indicative of the degree of absorption of said excitation radiation is generated. Then, the temporarily increased contact pressure is reduced while the wearable device remains secured to the body part of the subject.

Temporarily increasing the contact pressure may comprise controlling one or more actuators, e.g. to move an object such as the measurement body and/or an extendable member, and/or deforming a deformable member, e.g. by heating a bimetallic element and/or a shape-memory alloy structure, moving a bistable spring assembly from a first configuration to a second configuration, extending or contracting a pneumatically and/or hydraulically extendable member and/or inflating or deflating an inflatable body.

The method may comprise determining a thermal contact state and/or a pressure transmitting contact state between the contact surface of the measurement body and the skin of the subject, e.g. as detailed below for the second aspect of the invention. In some embodiments, the contact pressure may be increased temporarily in response to determining that there is no (or not sufficiently close) thermal contact and pressure transmitting contact based on the thermal contact state and/or the pressure transmitting contact state.

The analyte measurement may be performed as described above. In some embodiments, the method may also comprise an analyzing step, for example determining a measure of the concentration of the analyte in the tissue based on the response signal, e.g. as described above. Maintaining the temporarily increased contact pressure may comprise controlling one or more actuators, e.g. to maintain a position of an object such as the measurement body and/or an extendable member, and/or maintaining a shape or state of a deformable member, e.g. keeping an inflatable body in an inflated or deflated state.

Reducing the temporarily increased contact pressure may comprise partially or completely releasing the temporarily increased contact pressure, e.g. by reversing some or all of the measures taken for increasing the contact pressure. This may comprise controlling one or more actuators, e.g. to move an object such as the measurement body and/or an extendable member back to its initial position prior to increasing the contact pressure, and/or deforming a deformable member, e.g. to bring the deformable member back to its initial state or configuration prior to increasing the contact pressure.

Preferably, the method is executed automatically by the controller of the wearable device at least in part, e.g. by controlling the actuated means to temporarily increase the contact pressure, controlling the excitation radiation source and the detection device to perform the analyte measurement, controlling the actuated means to maintain the temporarily increased contact pressure and/or controlling the actuated means to reduce the temporarily increased contact pressure.

The method may further comprise determining the contact pressure between the measurement body and the skin of the subject, e.g. as described above. The response signal may be discarded for detection of the analyte if the determined contact pressure is below a (first) threshold, e.g. a pre-defined first threshold. In some examples, the contact pressure may be determined prior to performing the analyte measurement. The method may comprise increasing the contact pressure further in response to the determined contact pressure being below the first threshold or a below a second threshold different from the first threshold.

Instead of or in addition to the actuated means for temporarily increasing a contact pressure (e.g. for performing an analyte measurement), the wearable device may comprise means for monitoring a contact state between the contact surface of the measurement body and the skin of the subject, for example to determine a suitable point in time for performing an analyte measurement. When the wearable device is secured to the body part (i.e. while the subject wears the wearable device), the contact pressure between the measurement body and the skin may be increased temporarily, for example as a result of movements of the subject or the subject coming in contact with an external object, e.g. leaning against a wall, wherein the external object may press the wearable device and in particular the contact surface against said body part. By monitoring the contact state, the wearable device may determine whether the contact pressure is sufficiently high for reliably detecting said analyte and, if this is the case, may perform an analyte measurement in response. This provides an alternative way for reliably and accurately detecting an analyte in tissue of a human or animal subject by a method as set forth above that is implemented in a wearable device.

Therefore, according to a second aspect of the present invention, a wearable device for detecting an analyte in tissue of a human or animal subject is provided, wherein the wearable device comprises (1) a measurement body having a contact surface suitable to be brought in contact with a skin of the subject, said contact permitting heat and/or pressure waves generated by absorption of excitation radiation in the tissue to be transferred to said measurement body; (2) an excitation radiation source configured for irradiating excitation radiation at a plurality of wavelengths into the tissue to be absorbed by the analyte contained therein; (3) a detection device for detecting a physical response of the measurement body or of a component included therein to a heat and/or pressure wave received from the tissue upon absorption of the excitation radiation and for generating a response signal based on said detected physical response, said response signal being indicative of a degree of absorption of excitation radiation; (4) a controller to control the excitation radiation source to irradiate excitation radiation into the tissue to be absorbed by the analyte contained therein and to control the detection device to detect said physical response and to generate a response signal indicative of the degree of absorption of said excitation radiation; and (5) a holding portion for securing the wearable device to a body part, in particular a limb of the subject, said holding portion being configured to be arranged around the body part of the subject so as to extend circumferentially around the body part at least in part. The wearable device further comprises a contact monitor for monitoring a thermal contact state and/or a pressure transmitting contact state between the contact surface of the measurement body and the skin of the subject.

The measurement body, the excitation radiation source, the detection device, the controller and the holding portion may for example be embodied as detailed above for the wearable device according to the first aspect of the invention. The wearable device according to the second aspect of the invention may further comprise any combination of the features described above for the wearable device according to the first aspect of the invention, in particular some or all of the additional components described above such as the actuated means for temporarily increasing a contact pressure between the measurement body and the skin of the subject.

The thermal contact state and the pressure transmitting contact state may for example characterize whether there is thermal contact (i.e. contact permitting the transfer of heat waves) and pressure transmitting contact (i.e. contact permitting the transfer of pressure waves), respectively, between the contact surface and the skin. The thermal contact state and/or the pressure transmitting contact state may for example indicate whether there is a physical response of the measurement body or of a component included therein in response to irradiating excitation radiation into the tissue. Preferably, the thermal contact state and the pressure transmitting contact state may quantify the respective contact, e.g. characterize or quantify the contact pressure and/or an amount of attenuation of the respective waves upon transfer from the skin to the measurement body. The thermal contact state and/or the pressure transmitting contact state may in particular characterize whether the respective contact (for example the contact pressure) exceeds a threshold, e.g. a pre-defined threshold.

The contact monitor may be implemented in hardware, software or a combination thereof. In some embodiments, the contact monitor may be integrated into the controller at least in part.

The contact monitor may for example comprise one or more contact sensors, said one or more contact sensors comprising one or more of a pressure sensor configured to measure a pressure force between the measurement body and the skin of the subject (e.g. a capacitive pressure sensor or a piezoresistive strain gauge); an electrical sensor configured to measure an electric resistance and/or an electric capacitance and/or an electric voltage between the measurement body and the skin of the subject and/or to measure an inductance of an inductive element of the wearable device (e.g. an inductive element of the contact monitor); a humidity sensor configured to measure a humidity between the measurement body and the skin of the subject (e.g. at an interface and/or in the space between the contact surface and the skin); a fingerprint sensor configured to recognize a fingerprint on the contact surface of the measurement body; and an optical sensor configured to measure a reflection of light at the contact surface of the measurement body. In one embodiment, said optical sensor may be the detection device.

In some embodiments, said one or more contact sensors may comprise one or more auxiliary sensors as described above for the wearable device according to the first aspect, for example one or more of a blood pressure sensor, a pulse/heart rate sensor, an oxygen saturation sensor, a hydration sensor, a body temperature sensor, a breath rate sensor, a lactate sensor, a blood alcohol sensor, a carbon monoxide sensor, an urea sensor, a creatinine sensor, an albumin sensor, a bilirubin sensor and a hemoglobin sensor.

The contact monitor may be configured to determine the thermal contact state and/or the pressure transmitting contact state using said one or more contact sensors. The contact monitor may in particular be configured to determine whether there is a contact between the skin of the subject and the contact surface of the measurement body using said one or more contact sensors. Contact between the skin and the contact surface may for example be associated with an increase in pressure force, a decrease in electric resistance, a change in inductance, capacitance, voltage, humidity and/or reflected light intensity and/or with the fingerprint sensor being able to recognize the fingerprint. The contact monitor may for example be configured to determine whether there is a contact by comparing one or more of the aforementioned parameters and/or a rate of change thereof to a respective threshold.

Additionally or alternatively, the contact monitor may comprise a measurement module which is configured to detect, upon an activity of the excitation radiation source irradiating excitation radiation into the tissue, whether there is a physical response of the measurement body or of a component included therein, in particular by using the detection device. Said measurement module may be implemented in software and may in particular be provided as a software module of the controller. Detecting whether there is a physical response may for example comprise detecting whether there are one or more characteristic features associated with a heat and/or pressure wave generated by absorption of excitation radiation in the tissue being transferred to said measurement body. This may for example comprise detecting whether there is a change in the response signal (e.g. a peak or a dip) in response to one or more pulses of excitation radiation, for example whether the response signal contains contributions at or associated with a modulation frequency of the excitation radiation (e.g. whether said contribution exceeds a certain weight or threshold). In some embodiments, the measurement module may be configured to determine the contact pressure between the measurement body and the skin of the subject, for example as detailed above for the wearable device according to the first aspect, e.g. by performing a measurement at one or more wavelengths at which the degree of absorption of said excitation radiation in said tissue is substantially independent of a concentration of the analyte in the tissue.

In some embodiments, the contact monitor may be configured to generate an alarm (e.g. an acoustic alarm and/or an optical alarm) and/or to block the execution of an analyte measurement based on said thermal contact state and/or said pressure transmitting contact state. In some embodiments, the wearable device may comprise actuated means for temporarily increasing a contact pressure between the measurement body and the skin of the subject, e.g. as detailed above for the wearable device according to the first aspect of the invention. The contact monitor may be configured to activate the actuated means for temporarily increasing a contact pressure between the measurement body and the skin of the subject based on said thermal contact state and/or said pressure transmitting contact state. The contact monitor may in particular be configured to perform one or more of the aforementioned actions in response to determining that there is no contact between the skin of the subject and the contact surface of the measurement body and/or that contact between the skin and the contact surface is not sufficiently close (e.g. that the contact pressure is below a threshold).

According to the second aspect, the present invention further provides a method for detecting an analyte in tissue of a human or animal subject using a wearable device according to any one of the embodiments described herein, the method comprising securing the wearable device to a body part, in particular a limb of the subject such that the contact surface of the measurement body faces a skin of the subject; determining a thermal contact state and/or a pressure transmitting contact state between the contact surface of the measurement body and the skin of the subject; and in response to the thermal contact state and/or the pressure transmitting contact state indicating that there is thermal contact and pressure transmitting contact, respectively, between the contact surface of the measurement body and the skin of the subject, irradiating excitation radiation into the tissue to be absorbed therein using the excitation radiation source, detecting the physical response of the measurement body or a component included therein and generating a response signal indicative of the degree of absorption of said excitation radiation using the detection device (e.g. performing an analyte measurement).

The solution provided by the present invention is not limited to the detection assembly and the detection method as set forth above, but may also be applied to wearable devices having a different detection or sensor assembly and/or employing a different detection or sensing method, in particular contact-based sensors and/or contact-based detection or sensing methods. In some examples, the wearable device may thus not comprise some or all of the measurement body, the excitation radiation source, the detection device and the controller.

The present invention thus also provides a wearable device for performing a measurement on a human or animal subject (e.g. detecting an analyte and/or measuring a physiological parameter or quantity such as a pulse, an oxygen saturation and/or an electrocardiogram), in particular on tissue of the subject such as a skin of the subject. The wearable device comprises a sensor for performing the measurement (e.g. an optical measurement and/or an electrical measurement) and a holding portion for securing the wearable device to a body part, in particular a limb of the subject. The sensor has a contact surface suitable to be brought in contact with the skin of the subject (e.g. in electrical contact, thermal contact and/or pressure transmitting contact), wherein the contact surface may be involved in the measurement (for example by transmitting an electrical signal (e.g. a current), an optical signal (e.g. an incident beam of light and/or light reflected or scattered by tissue of the subject) and/or a heat and/or pressure wave). The wearable device further comprises actuated means for temporarily increasing a contact pressure between the sensor, in particular the contact surface of the sensor, and the skin of the subject by reversibly performing one or more of reducing an inner diameter of the wearable device, reducing an inner circumference of the wearable device and moving the sensor or a part thereof, in particular the contact surface, radially inward towards the body part, e.g. as described above.

Said wearable device for performing a measurement on a human or animal subject may further comprise any combination of features as described above for the wearable device for detecting an analyte in tissue of a human or animal subject, in particular some or all of the components of the wearable device for detecting an analyte in tissue of a human or animal subject as set forth above.

SHORT DESCRIPTION OF THE FIGURES

In the following, a detailed description of the invention and exemplary embodiments thereof is given with reference to the figures. The figures show schematic illustrations of

Fig. 1: the measurement principle underlying embodiments of the invention;

Fig. 2: the absorption spectrum of glucose in water, with the water background subtracted;

Fig. 3: a sectional view of an apparatus suitable for carrying out embodiments of the invention relying on response signals based on a deflection of a detection light beam;

Fig. 4: results of a Clarke's error grid analysis obtained with an apparatus of the type shown in Fig. 3;

Fig. 5: an apparatus suitable for carrying out embodiments of the invention relying on response signals based on piezoelectric responses to heat or pressure waves received from the tissue subjected to the analysis;

Fig. 6: an apparatus suitable for carrying out embodiments of the invention relying on response signals based on interferometrically detected phase changes in a detection light beam;

Fig. 7a, 7b: a wearable device for detecting an analyte in tissue of a human or animal subject according to an exemplary embodiment of the invention;

Fig. 8a, 8b: a main housing of a wearable device for detecting an analyte in tissue of a human or animal subject in accordance with an exemplary embodiment of the invention;

Fig. 9a, 9b: wearable devices for detecting an analyte in tissue of a human or animal subject comprising a holding portion with a plurality of segments according to an exemplary embodiment of the invention;

Fig. 10: a wearable device for detecting an analyte in tissue of a human or animal subject comprising a removable battery in accordance with an exemplary embodiment of the invention;

Fig. 11: a flow chart of a method for detecting an analyte in tissue of a human or animal subject using a wearable device according to an exemplary embodiment of the invention; and

Fig. 12: a wearable device for detecting an analyte in tissue of a human or animal subject comprising a contact monitor according to an exemplary embodiment of the invention.

DESCRIPTION OF THE PREFERRED EMBODIMENTS

It is to be understood that both the foregoing general description and the following description are exemplary and explanatory only and are not restrictive of the methods and devices described herein. In this application, the use of the singular may include the plural unless specifically state otherwise. Also, the use of "or" means "and/or" where applicable or unless stated otherwise. Those of ordinary skill in the art will realize that the following description is illustrative only and is not intended to be in any way limiting. Other embodiments will readily suggest themselves to such skilled persons having the benefit of this disclosure. Reference will now be made in detail to various implementations of the example embodiments as illustrated in the accompanying drawings. The same reference signs will be used to the extent possible throughout the drawings and the following description to refer to the same or like items.

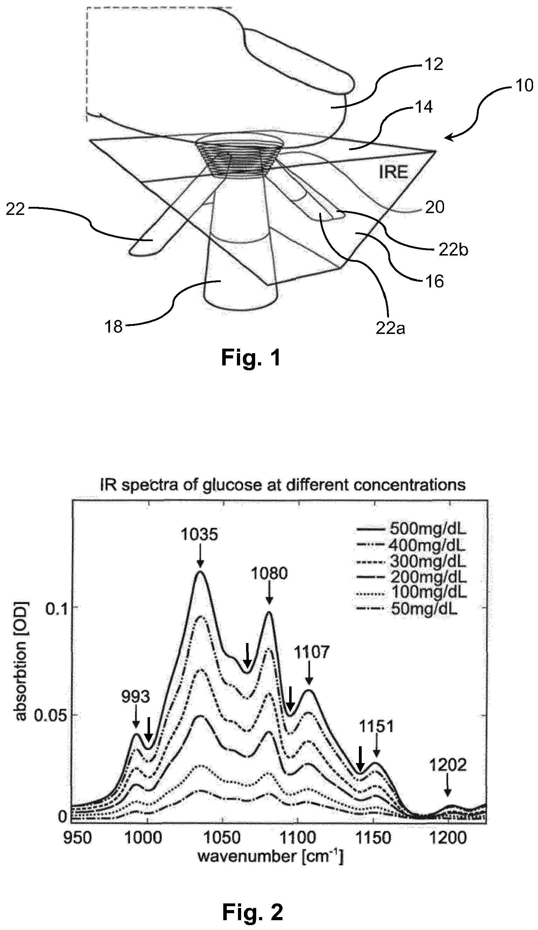

Fig. 1 is a schematic illustration of the measurement principle underlying the analyte measurement procedure summarized above and described in more detail in the following. While the device and method of the invention are suitable for analyzing various types of tissue of both human and animal subjects comprising at least one analyte, the following description will focus on specific embodiments, where the tissue is the skin of a patient and the analyte is glucose within the interstitial fluid of the skin. It is to be understood that all details and explanations given in the following with specific reference to glucose measurement are considered in relation to other types of tissue and analytes as well, where applicable, without explicit mention in the following.

In the illustration of Fig. 1, a fingertip 12 of a user is brought in thermal contact with a contact surface 14 of a measurement body 16. In an alternative implementation, which is not shown, the fingertip may be coupled acoustically to the measurement body through an acoustic cell which may include a hollow space filled with a liquid or gas allowing for a transfer of pressure waves to the measurement body.

An excitation beam 18 is irradiated to the measurement body 16 through air or through a waveguide (not shown) and then through the measurement body 16 and into the skin at the fingertip 12. In order to determine the concentration of the glucose in the skin, in particular in the interstitial fluid of the skin, various wavelengths of the excitation radiation 18 are chosen one after the other or at least partially at the same time for absorption measurement, such that from the measured absorption values the concentration of the glucose can be determined.

In Fig. 2, absorption spectra are shown for different concentrations of glucose in water, wherein the contribution of the absorption by water has been subtracted. As is seen therein, the glucose molecule has several characteristic absorption peaks in the mid infrared region at wave numbers ranging between 993 $cm^{-1}$ and 1202 $cm^{-1}$, corresponding to wavelengths ranging from 10.07 $\mu$m to 8.32 $\mu$m, respectively. In between adjacent absorption peaks, local absorption minima are seen, which are indicated by vertical arrows without wave numbers in Fig. 2. As is apparent from Fig. 2, particularly the difference in absorption at the absorption peaks and the local absorption minima are characteristic for the glucose concentration. Accordingly, in order to be able to determine the glucose concentration, it is preferable to measure the absorption at some or all of the absorption peaks and at some or all of the local absorption minima and potentially also at some point between the maxima and minima. These wavelengths are referred to as a "analyte(glucose)-characteristic wavelengths" herein. Note, however, that the absorptivity at the lowest local minimum at 1140 cm-1 is still 18% of the absorptivity at the highest peak at 1035 cm-1 in the relevant part of the spectrum. Accordingly, the absorption at any of these wavelengths will noticeably depend on the concentration of the glucose, such that these wavelengths are characteristic for the glucose, i.e. "glucose-characteristic wavelengths". In contrast to this, at about 1180 cm-1, the absorptivity is practically zero, and hence a global rather than a local minimum, and this wavelength is obviously not characteristic for the glucose (but may e.g. be used to determine a contact pressure between the measurement body 16 and the skin of the fingertip 12). While wavelengths exactly at the absorption peaks or local absorption minima are the preferred choices for the glucose-characteristic wavelengths, wavelengths close to the peaks/local minima but in an individually defined distance from them may also be used. Accordingly, as understood herein, "analyte-characteristic wavelengths" are also wavelengths where the difference in absorption to that at the closest absorption peak or the closest local absorption minimum is less than 30%, preferably less than 20% of the difference in absorption between the closest absorption peak and closest local absorption minimum.

The intensity of the excitation beam 18 is time modulated with a certain frequency f, such that the excitation radiation, in this case excitation light, has alternating intervals of high intensity and low or even vanishing intensity. Without wishing to limit the modulation to any particular waveform, high intensity intervals are referred to as "excitation light pulses" in the following. During the excitation light pulses, excitation light having the glucose-characteristic wavelength will be absorbed, such that the radiation energy will be converted to heat. Since the glucose molecules relax from the excited state within approximately $10^{-12}$ s, the generation of a corresponding heat pulse and/or pressure wave can be regarded as occurring instantaneously for all practical purposes.

Accordingly, along with the excitation light pulses, local heat pulses are generated at the absorption site, leading to a temperature field that varies as a function of space and time and that could be referred to as a thermal wave. As was explained above, the term thermal "wave" is somewhat misleading, since the travel of heat through the material is not governed by a wave equation, but by a diffusion equation instead. However, the notion of a "heat wave" is correct at least to the extent that heat pulses propagate from within the skin to the surface 14 of the measurement body 16 and into the measurement body 16 similarly to what one is used to from wave propagation. A thermal gradient 20 that is caused by such a heat pulse is schematically shown in Fig. 1.

The heat received by the measurement body 16 from the skin of the fingertip 12 causes a physical response that can be detected with one of various possible detection devices which are devised for generating a response signal based on the physical response, wherein this response signal is indicative of the degree of absorption of the excitation light. Various ways of detecting the physical response and generating suitable response signals will be described below.

However, irrespective of the precise way of detecting the physical response, it is worth noting that the maximum depth underneath the surface of the skin in which the absorption can be detected by means of heat pulses travelling to the measurement body 16 is found to be limited to a good approximation by the thermal diffusion length $\mu_t$ of the skin, which is defined as

$$\mu t(f) = \sqrt{\frac{k_t}{\rho \cdot C_p \cdot 2\,f}}$$

and which depends on the density p, the specific heat capacity $C_p$, and the thermal conductivity $k_t$ of the tissue as well as on the modulation frequency f of the excitation light. In other words, by selecting the modulation frequency f, a depth can be defined up to which any absorption of the excitation light is reflected in the heat pulses received at the measurement body 16.

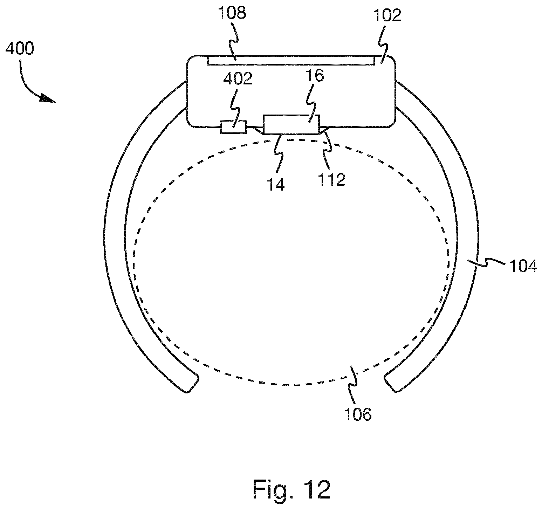

With reference again to Fig. 1, in the embodiment shown, the physical response to the absorption heat received from the skin is a change in refractive index in an area close to the surface 14 of the measurement body 16 where the heat gradient 20 is transiently formed. This local change in refractive index forms what could be regarded as a thermal lens that can be detected by means of a detection light beam 22. The detection beam 22 is passing through the thermal lens or heat gradient region and then reflected at the interface of the measurement body 16 and the skin of the Fig. 12. Whenever a heat pulse is received from the skin, a local change in refractive index occurs, and this leads to a deflection of the detection beam 22 by the interaction with the material of the measurement body in the region of the thermal lens. In Fig. 1, reference sign 22b corresponds to the non-deflected detection beam 22, whereas reference sign 22a corresponds to the detection beam when it is deflected due to the thermal lens formed in the heat gradient region 20. This deflection can be measured and forms an example of the aforementioned response signal. The degree of the deflection is indicative of the amount of heat received, and hence the degree of absorption of the excitation light 18 in the skin of the finger 12.

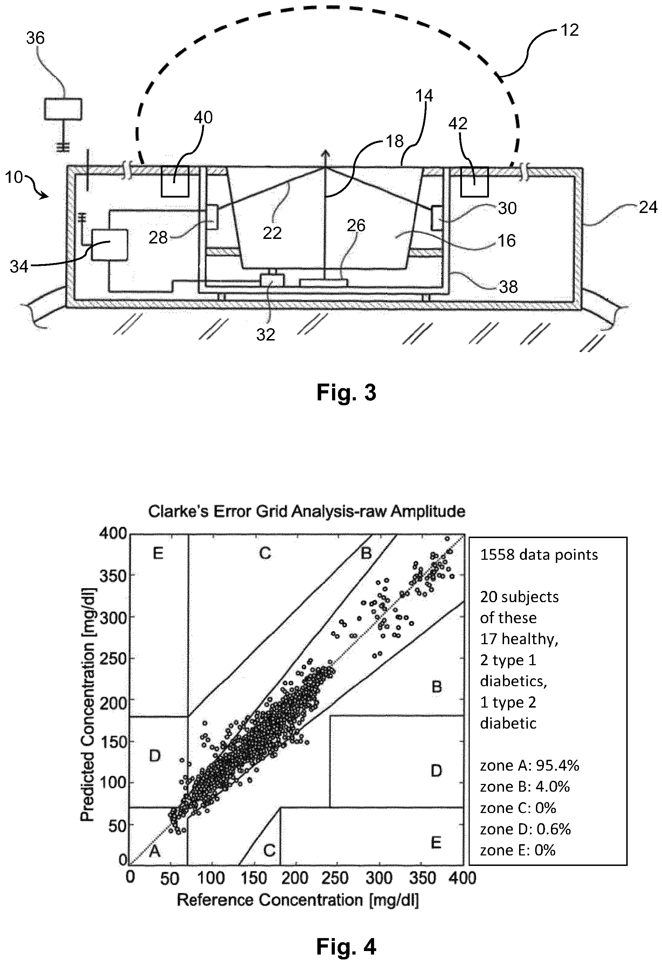

Fig. 3 shows a more detailed sectional view of an apparatus 10 that relies on the measurement principle as illustrated with reference to Fig. 1. The apparatus 10 comprises a housing 24 which includes the measurement body 16, having a top surface (contact surface) 14 on which a body part of the subject/patient such as a finger 12 rests. Within the housing 24, an excitation light source (excitation radiation source) 26 is provided, which generates the excitation light beam 18. In the embodiment shown, the excitation light source 26 comprises an array of quantum cascade lasers (not shown) each having a dedicated wavelength. For example, the array of quantum cascade lasers could include individual quantum cascade laser elements with wavelengths corresponding to the absorption peaks and local minima shown in Fig. 2 (i.e. the glucose-characteristic wavelengths), as well as other wavelengths that can be used for reference measurements (e.g. for determining a contact pressure between the measurement body 16 and the skin of the fingertip 12, for example one or more characteristic wavelengths of water), or for detecting other substances that could be disturbing to the measurement of the glucose, for example lactate or albumin.

The apparatus 10 further comprises a light source 28, for example a laser, for emitting the detection beam 22, as well as a position-sensitive detector or sensor 30 (e.g. a quadrant photodiode) which allows for detecting the deflection of the detection beam 22. The light source 28 and the position-sensitive detector 30 together form an example of a detection device for detecting the physical response of the measurement body 16 to heat and/or pressure waves received from the fingertip 12. The measurement body 16 in this case is transparent for both, the excitation light beam 18 as well as the detection light beam 22.

In addition, a camera 32 or another imaging device is provided that allows for taking images of the contact surface 14 of the optical medium 16, to thereby record a skin pattern such as a fingerprint of the finger 12 resting on the contact surface 14. This skin pattern can be processed by a control unit 34 such as to identify and/or authenticate a user via his or her skin pattern (e.g. fingerprint).

The control unit 34 also serves for controlling the light sources 26 and 28 for the excitation light and the detection light, respectively, as well as the sensor 30. The control unit 34 forms an example of a "controller" as referred to above. The control unit 34 may also be in wireless connection with an external data processing device 36 to exchange data. For example, via the wireless connection, user-specific calibration data can be retrieved by the control unit 34 for the user that is identified via the fingerprint. In some examples, the control unit 34 and the external data processing device 36 may together form an example of a "controller" as referred to above. The controller can be comprised by one or more processors, microcontrollers, computers, ASICs, FPGAs, or the like. The controller may be distributed, as indicated in Fig. 3, with various components in data communication with each other, or could be formed by a single control unit, such as the control unit 34, which would be devised for all of the control functionalities described herein. The controller may be generally embodied in hardware, in software, or a combination of both.

As is further seen in Fig. 3, the excitation and detection light sources 26 and 28, as well as the position sensitive detector 30 are all attached to a common carrier structure 38. This means that these components can be preassembled with precision on this structure 38, such that they do not need to be individually adjusted or calibrated when assembling the apparatus 10.

In addition, the apparatus 10 comprises a corneometric device 40 that allows for measuring the water content of the skin. Corneometric devices for measuring the water content in the upper layer of the skin are per se known in the art and need not be described in detail here. For example, known corneometric devices measure the impedance, in particular capacitive impedance of the skin using two interdigital electrodes to which an AC voltage is applied. The corneometric device 40 of Fig. 3 is in contact with the fingertip 12 when the latter rests on the contact surface 14 of the measurement body 16. The corneometric device 40 is an example of the aforementioned "auxiliary sensors", i.e. a sensor that is per se unrelated to the measurement apparatus for measuring the analyte absorption.

The apparatus also comprises a pH-sensor 42 for measuring the pH value of the skin. pH sensors for measuring the pH value on surfaces, including those of the skin are per se known from prior art and need not be described in detail herein. pH sensors for measuring the pH value of the skin are commercially available for medical but also for cosmetic purposes.

Fig. 4 shows results of a Clarke's error grid analysis obtained with an apparatus of the type shown in Fig. 3, illustrating that with the measurement procedure described with reference to Fig. 1 to 3, indeed very reliable blood sugar concentrations can be measured in a purely non-invasive manner. The data shown in Fig. 4 are taken from WO 2017/09782 A1 and do not yet reflect improvements of the present invention. The present invention allows for obtaining results of similar or better quality even with wearable devices, for which the conditions in which the measurement procedure is performed are more challenging to control than for a stationary apparatus (e.g. a dedicated stand-alone device) that is for example employed in a clinical setting.

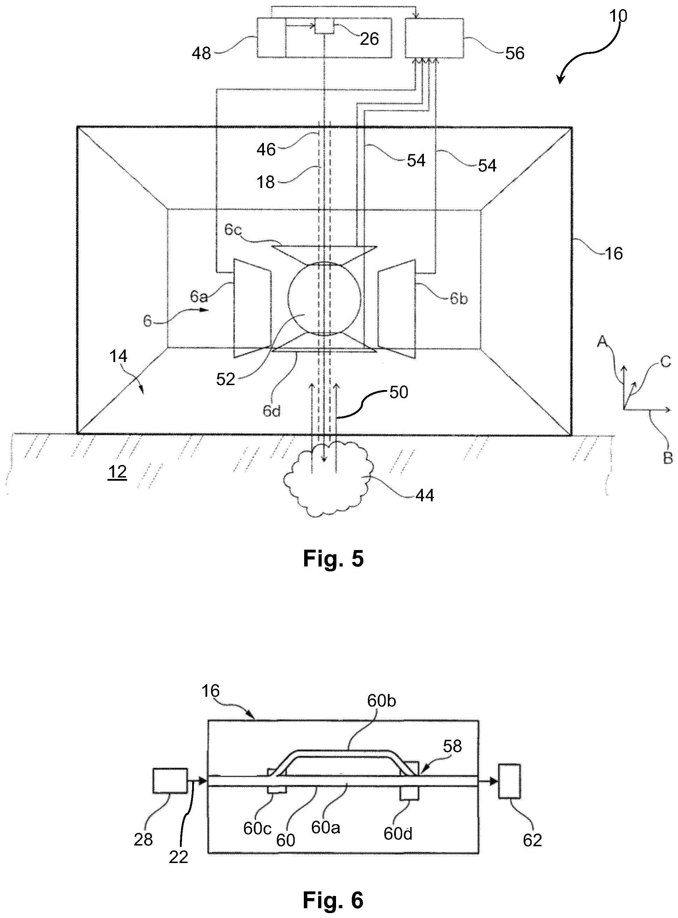

Fig. 5 schematically shows an apparatus 10 which relies on the same general principle involving absorption of excitation radiation for generating heat pulses received by the measurement body 16 from the tissue of the fingertip/body part 12 as that of Fig. 1 and 3, but differs in the physical response exploited and in the way the corresponding response signals are generated. Such an apparatus 10, as well as a large number of variations thereof are described in detail in WO 2019/11059782 incorporated herein by reference, such that a detailed description may be omitted herein. As before, the apparatus comprises a measurement body 16 having a surface 14 which is brought in contact or coupling with the skin of a finger 12. Also, a source 26 for an excitation light beam 18 with modulated intensity is provided, which is irradiated into a region 44 underneath the surface of the skin 12 and absorbed therein. In this embodiment, the excitation light beam 18 runs through a bore 46 indicated by hashed lines through the measurement body 16, such that the measurement body 16 itself need not be transparent for it.

A control unit 48 is provided for modulating the intensity of the excitation light beam 18. This can generally be done in various ways, including a mechanical chopper or an element having a transmissivity or reflectivity that can be electronically controlled. However, in preferred embodiments, the intensity is modulated by modulating the on/off times of the excitation light source 26 as well as the operating current during the on-times thereof. The control unit 48 may for example be integrated into a controller such as the control unit 34 of Fig. 1 at least in part.

A thermal wave caused by the time varying absorption of the intensity modulated excitation beam 18 in the region 44 of the skin 12, which is symbolically represented by arrows 50, enters the measuring body 16 where it can be detected in a detection region 52 which has piezoelectric properties. Pressure changes associated with received heat 50 or pressure waves lead to electrical signals that can be recorded with electrodes 6a to 6d, which are connected via conducting leads 54 with an evaluation device 56 for analyzing the tissue (the skin of figer 12). The electrodes 6a to 6d, in some examples together with the evaluation device 56, may form an example of a detection device as referred to above. The evaluation device 56 maybe a digital processing device, for example a microcontroller or processor or a computer. In some exmaples, the evaluation device may be integrated into a controller such as the control unit 34 of Fig. 1 at least in part. In this example, the change in pressure resembles the physical response of the measurement body 16, or other component included therein, to heat received from the tissue of the fingertip 12 upon absorption of the excitation radiation, which is detected using the piezoelectric properties of the measurement body 16 and the electrodes 6a to 6d, and which leads to electrical signals representing the response signal that is indicative of the degree of absorption of excitation radiation 18.

In alternative variants suggested by the present applicant, as for example disclosed in international application PCT/EP2019/064356 included herein by reference, the detection device may comprise an interferometric device allowing for assessing said change in phase of a first part of the detection beam with respect to a second part of the detection beam, wherein only one of the parts of the detection beam passing through a measurement arm is affected by the effects of the heat or pressure wave in the measurement body 16, and generates a response signal indicative of said change in phase. In this example, the physical response of the measurement body 16 (or a component included therein) to heat received from said material 12 upon absorption of said excitation radiation 18 is again a local change in index of refraction, while the response signal is in this case an interferometric signal reflecting a change in the phase of the detection beam due to the local change in refractive index.

This is schematically illustrated in Fig. 6, where a measurement body 16 is shown which is to be brought in contact with the skin of a body part (such as the finger, not shown in Fig. 6). In this example, the measurement body 16 may be a silicon substrate in which a light guiding structure 58 is provided, which forms an interferometric device 60. The interferometric device 60 forms a Mach-Zehnder interferometer, having a measurement arm 60a and a reference arm 60b. Detection light 22 generated by a detection light source 28 is fed into the light guiding structure 58 and is splitted by a splitter 60c into a portion or a part of the detection beam travelling along the measurement arm 60a and a portion or part travelling along the reference arm 60b, which portions are then united by a combiner 60d. The measurement body 16 is used or arranged such that the reference arm 60a is exposed to heat received from the skin upon absorption of excitation light, but not, or at least to a much lesser extent, the reference arm 60b. Due to received heat, the refractive index in the measurement arm 60a will change, which in turn leads to a phase shift of the detection light 22 travelling along the measurement arm 60a. Since the light travelling along the reference arm 60b is unaffected by the heat received, there will be a change in relative phase of the two portions of light combined by the combiner 60d, which leads to an interference pattern that can be detected using a detector 62. Accordingly, the light source 28, the interferometric device 60 and the detector 62 may together form an example of a detection device as referred to above.

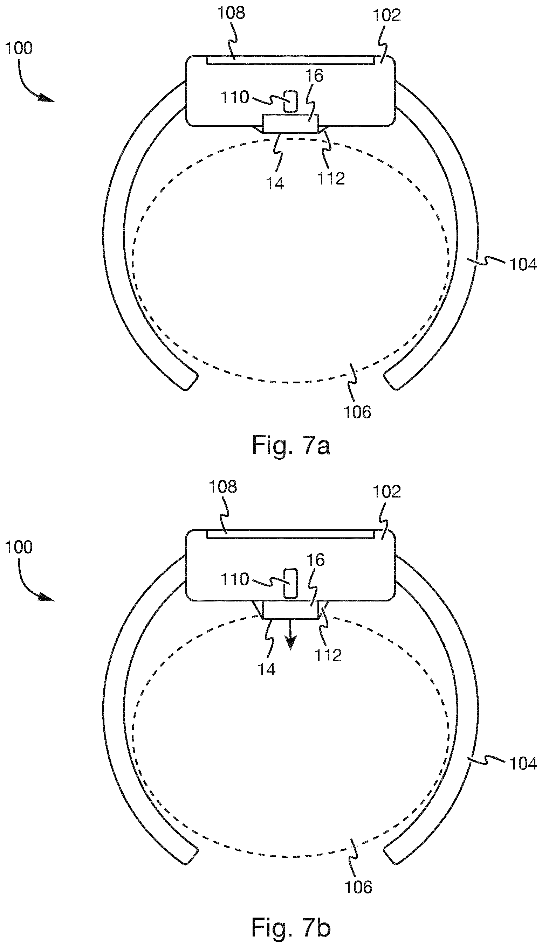

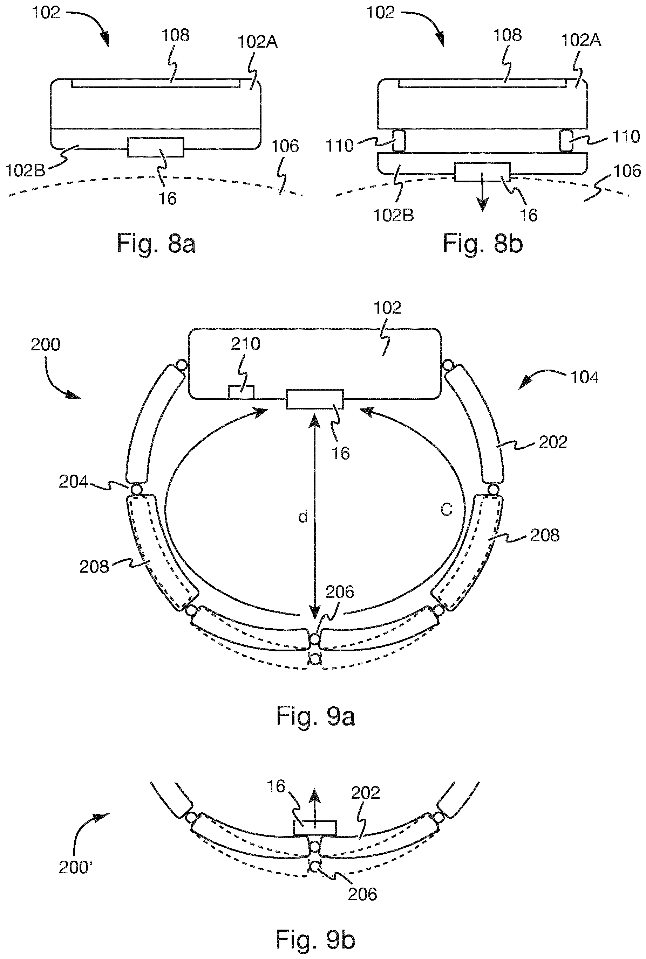

Figs. 7a and 7b show a schematic illustration of a wearable device 100 for detecting an analyte in tissue of a human or animal subject according to an exemplary embodiment of the invention. The wearable device 100 comprises a main housing 102 and a holding portion 104 for the securing the wearable device 100 to a body part 106 of a human or animal subject, for example to an upper arm or a wrist of a user/patient. The holding portion 104 is configured to be arranged around the body part 106 so as to extend circumferentially around the body part 106 at least in part as illustrated in Figs. 7a, 7b or completely as e.g. shown in Fig. 9a. The holding portion 104 may for example be a wristband or a similar structure. The wearable device 100 further comprises a display 108, which is arranged on or in an upper surface or upper wall of the main housing. In some examples, the wearable device 100 may be a smartwatch.

In the following, the direction extending azimuthally around the body part 106 is referred to as the circumferential direction and a direction that is perpendicular to the surface (i.e. the skin) of the body part 106 is referred to as a radial direction.

The wearable device further comprises an apparatus for detecting the analyte (e.g. glucose) in tissue of the body part 106, e.g. in the skin, using a detection method as set forth above. The apparatus for detecting the analyte is arranged in the main housing 102 and may for example be similar to the apparatus of any one of Figs. 3, 5 and 6. In particular, the apparatus comprises a measurement body 16 having a contact surface 14 that is suitable to be brought in contact with the skin of the subject wearing the wearable device 100 to permit heat and/or pressure waves generated by absorption of excitation radiation in said tissue of the body part 106 to be transferred to the measurement body 16. The apparatus further comprises an excitation radiation source (not shown) for irradiating excitation radiation at a plurality of analyte-characteristic wavelengths into the tissue, a detection device (not shown) for detecting a physical response of the measurement body 16 to heat and/or pressure waves received from the tissue upon absorption of the excitation radiation and for generating a response signal based on the detected physical response as well as a controller (not shown) for controlling the excitation radiation source and the detection device to perform analyte measurements. The measurement body, the excitation radiation source, the detection device and the controller may for example be embodied as described above with reference to Figs. 3, 5 and 6. In one example, the main housing 102 is similar to or corresponds to the housing 24 of the apparatus 10 of Fig. 3. In some examples, the controller maybe integrated into a main control unit of the wearable device 100 at least in part, wherein the main control unit may for example control the display 108 and may be configured to provide smartwatch capabilities as known in the art.

The wearable device 100 further comprises actuated means for temporarily increasing a contact pressure between the measurement body 16 and the skin of the subject (i.e. the skin of the body part 106). In the example of Figs. 7a, 7b, the measurement body 16 is arranged in a bottom surface or bottom wall of the main housing 102 (i.e. opposite to the display 108) such that the measurement body 106 protrudes from the bottom surface or wall. When the wearable device 100 is secured to the body part 106, the contact surface 14 faces the body part 106. An interface between the bottom surface or wall and the measurement body 106 may be covered or sealed off by a sealing 112, e.g. a rubber sealing, which may for example cover or seal off exposed portions of the sidewalls of the measurement body 106. The actuated means comprise an actuator 110, for example an electric actuator or a piezoelement, that is configured to move the measurement body 16 back and forth in the radial direction, i.e. radially inward towards the body part 106 and radially outward away from the body part 106 (i.e. down and up, respectively, in the example of Figs. 7a, 7b).

In Fig. 7a, the wearable device 100 is depicted in a normal state, in which the contact pressure between the measurement body 16 and the skin of the subject is not increased (which may e.g. be the state in which the wearable device 100 is usually worn by the subject). Fig. 7b depicts the wearable device 100 in a measurement state, in which the contact pressure between the measurement body 16 and the skin of the subject is increased temporarily by moving the measurement body 16 towards the body part 106 with the actuator 110 (i.e. such that the measurement body 16 protrudes from the main housing 102 by a larger distance than in the normal state). This may allow for pressing the measurement body 16 against the body part 106 to ensure a tight fit and close contact between the measurement body 16 and the skin of the subject for performing an analyte measurement. The actuator 110 may for example be configured to move the measurement body 16 along the radial direction by up to 0.5 mm, preferably by up to 1 mm, in some examples by up to 2 mm, in one example by up to 5 mm.

Figs. 8a, 8b depict a schematic illustration of a main housing 102 of a wearable device for detecting an analyte in tissue of a human or animal subject in accordance with an exemplary embodiment of the invention. The main housing 102 may be used in a wearable device according to any one of the embodiments described herein, for example as the main housing of the wearable device 100 of Figs. 7a, 7b. The main housing 102 comprises an upper portion 102A and a lower portion 102B, wherein the upper portion 102A is to face away from the body part 106 of the subject, while the lower portion 102B is to face towards the body part 106 when the wearable device is secured to the body part 106. A display 108, which may e.g. be a liquid-crystal display and in particular an OLED display, is arranged in or on the upper portion 102A. The upper portion 102A may further comprise the controller (not shown) for controlling the excitation radiation source and the detection device as described above, wherein the excitation radiation source and the detection device or components thereof may be arranged in either one or both of the upper portion 102A and the lower portion 102B. The measurement body 16 with the contact surface is arranged in the lower portion 102B, e.g. such that the measurement body 16 protrudes from a bottom surface of the lower portion 102B as shown in Figs. 8a, 8b.

The main housing 102 comprises actuated means for temporarily increasing the contact pressure between the measurement body 16 and the skin of the subject. The lower portion 102B with the measurement body 16 is mounted movably with respect to the upper portion 102A such that the lower portion 102B can be moved in the radial direction, e.g. away from the upper portion 102A towards the body part 106 and vice-versa. In Fig. 8a, the main housing 102 is depicted in a normal state, in which the lower portion 102B is in contact with the upper portion 102A and the contact pressure between the measurement body 16 and the skin of the subject is not increased. Fig. 8b depicts the main housing 102 in a measurement state, in which the lower portion 102B has been displaced from the upper portion 102A (e.g. such that the lower and upper portions 102B, 102A are separated by a gap) and the contact pressure between the measurement body 16 and the skin of the subject is increased.

For temporarily increasing the contact pressure, the actuated means comprises a pair of actuators 110 that are configured to move the lower portion 102B, e.g. via a movable shaft or piston connected between the upper portion 102A and the lower portion 102B. Additionally or alternatively, the actuated means may also comprise one or more deformable members (not shown) that are configured to be deformed, extended and/or retracted reversibly to move the lower portion 102B relative to the upper portion 102A. For example, an inflatable body (not shown) such as a fluid-filled cushion may be arranged between the lower portion 102B and the upper portion 102A. The inflatable body may be configured to receive a fluid such as air for extending the inflatable body from a compressed state (which may e.g. correspond to the normal state) to an expanded state (which may e.g. correspond to the measurement state).

Fig. 9a shows a schematic illustration of a wearable device 200 for detecting an analyte in tissue of a human or animal subject according to an exemplary embodiment of the invention. The wearable device 200 comprises a main housing 102 and a holding portion 104. The main housing 102 may contain the components for performing analyte measurements as set forth above including the measurement body 16 and may be embodied as described above. The main housing 102 may for example be similar to the main housing 102 of the wearable device 100 of Figs. 7a, 7b or to the main housing 102 of Fig. 8a, 8b. In some examples, the main housing may not comprise an actuator for moving the measurement body 16.

The holding portion 104 is configured for securing the wearable device 200 to a body part (not shown) of the subject. The holding portion 104 comprises a plurality of segments 202 that are connected with each other via a plurality of hinges 204 to form a chain, which may e.g. be configured to be placed around an upper arm or a wrist of the subject. The holding portion 104 comprises actuated means for temporarily increasing a contact pressure between the measurement body 16 and the skin of the subject. In Fig. 9a, the dashed lines illustrate the position and shape of the segments 202 in the normal state without increasing the contact pressure, whereas the solid lines illustrate the position and shape of the segments 202 in the measurement state, in which the contact pressure is increased temporarily.

The actuated means comprise an actuated hinge 206 arranged between a pair of adjacent segments 202. In the example of Fig. 9a, the actuated hinge 206 is arranged in a portion of the wearable device 200 that is opposite to the main housing 102 and thus to the measurement body 16. The actuated hinge 206 is configured to rotate the pair of adjacent segments 202 with respect to each other to move the actuated hinge 206 radially inwards towards the body part, e.g. by increasing an angle enclosed by the adjacent segments 202 as illustrated in Fig. 9a. Thereby, the actuated hinge 206 can reduce an inner diameter d of the wearable device 200, e.g. the distance between the actuated hinge 206 and the main housing 102. The actuated hinge 206 may for example comprise an actuator (not shown) for rotating the pair of adjacent segments 202. Additionally or alternatively, the actuated hinge 206 may comprise a deformable member (not shown) such as a bimetallic coil, a shape-memory alloy structure or a bistable spring assembly that is configured to bring the actuated hinge 206 from a first configuration (e.g. the normal state in which the adjacent segments enclose a first angle) to a second configuration (e.g. the measurement state in which the adjacent segments enclose a second angle that is larger than the first angle, e.g. by up to 5°, in some examples by up to 10°, in one example by up to 20°). In some examples, the holding portion 104 may comprise a plurality of actuated hinges. For example, all of the hinges 204 may be actuated.

The actuated means further comprise two inflatable segments 208, e.g. segments that contain an inflatable body (not shown) such that the respective segment can be inflated from a compressed state (dashed lines) to an expanded state (solid lines). This may for example be achieved by pumping ambient air into the inflatable body with a fluid pump (not shown). When inflating the inflatable segments 208, a radial extent (e.g. a width along the radial direction) of the inflatable segments 208 may increase. Thereby, the inner diameter d between opposing portions of the wearable device 200 as well as an inner circumference C of the wearable device 200 (i.e. the length measured along inner surface of the main housing 102 and the holding portion 104 around the body part) can be reduced reversibly in order to temporarily increase the contact pressure between the measurement body 16 and the skin of the subject. In some examples, all of the segments 202 of the holding portion 104 may be inflatable. In some embodiments, the wearable device 200 may only comprise one of actuated hinges and inflatable segments. In one example, the inflatable segments 208 may be configured such that when the inflatable body arranged therein is deflated, e.g. by removing fluid therefrom, a circumferential extent (e.g. a length along the circumferential direction around the body part) decreases, e.g. to reduce the inner circumference of the wearable device 200.

The wearable device 200 further comprises at least one auxiliary sensor 210. The auxiliary sensor 210 may for example be an optical pulse and/or oxygen saturation sensor as known in the art. The auxiliary sensor 210 may e.g. comprise one or more light sources (not shown) for illuminating the skin of the subject and one or more photodetectors (not shown) for detecting light scattered or reflected from the body part, which may for example be used for determining a pulse of the subject and/or an oxygen saturation of blood of the subject. The auxiliary sensor 210 may be arranged on or in a bottom surface or bottom wall of the main housing 102, e.g. adjacent to the measurement body 16, so as to face the body part when wearable device 200 is secured thereto. The sensor signal of auxiliary sensor 210, which may e.g. be indicative of an amount of light scattered or reflected from the body part (and may thus e.g. allow for obtaining a pulse time trace or a pulse of the subject), may depend on the contact pressure between the measurement body 16 and the skin of the subject. For example, an amplitude of the sensor signal may increase when the contact pressure is increased. This may allow for determining the contact pressure between the measurement body 16 and the skin of the subject based on the sensor signal of the auxiliary sensor 210, e.g. based on a predetermined calibration curve and/or using a classifier such as a neural network-based classifier.

Additionally or alternatively, the contact pressure may also be determined by performing a measurement with the excitation radiation source and the detection device as set forth above, wherein the excitation radiation is generated at one or more wavelengths at which the degree of absorption of the excitation radiation in the tissue is substantially independent of the concentration of the analyte contained therein, e.g. at one or more characteristic wavelengths of water. At these wavelengths, a degree of absorption and/or the physical response of the measurement body may depend on the contact pressure (the degree of absorption and/or the physical response may e.g. larger the larger the contact pressure is) and may thus allow for determining the contact pressure.

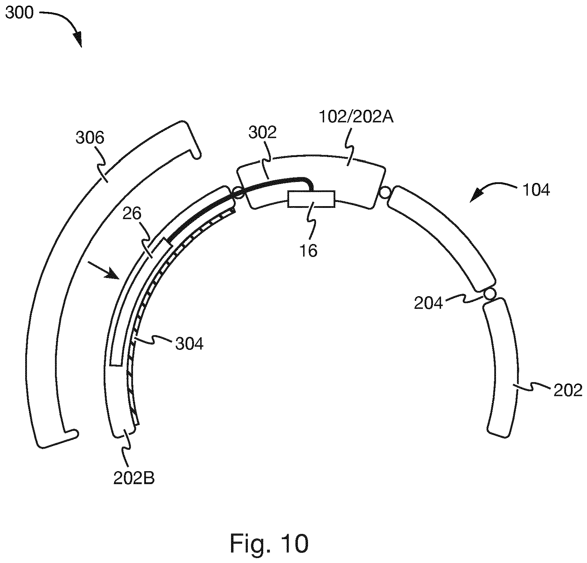

Fig. 9b depicts an alternative embodiment 200' of the wearable device 200 of Fig. 9a. In this example, the measurement body 16 is not arranged in the main housing 102, but along the holding portion 104 opposite to the main housing 102 in the vicinity of the actuated hinge 206. The measurement body 16 may for example be mounted on and/or in one or both of the adjacent segments 202 connected by the actuated hinge 206. This may for example allow for positioning the measurement body 16 on an inner side of the wrist of the subject, which may be advantageous for the analyte measurements. The detection device (not shown) and the excitation radiation source (not shown) or parts thereof may also be arranged outside of the main housing 102 in the vicinity of the measurement body 16, e.g. in and/or on one or both of the segments 202 connected by the actuated hinge 206. Additionally or alternatively, the detection device and/or the excitation radiation source or parts thereof may also be arranged in and/or on the main housing 102 and may for example be connected to the measurement body 16 by one or more waveguides and/or wires, e.g. as described below with reference to Fig. 10.

Fig. 10 shows a schematic illustration of a wearable device 300 for detecting an analyte in tissue of a human or animal subject in accordance with an exemplary embodiment of the invention. The wearable device 300 also comprises a main housing 102, which may contain some or all of the components (e.g. at least the controller (not shown) and the measurement body 16) for performing analyte measurements as set forth above as well as a display (not shown). The wearable device 300 further comprises a holding portion 104 with a plurality of segments 202 for securing the wearable device 300 to a body part (not shown) of the subject. The main housing 102 may form one of the segments 202 of the holding portion 104 as illustrated in Fig. 10, which may also be referred to as the first segment 202A in the following.

In the example of Fig. 10, the excitation radiation source 26, which may for example be an array of quantum cascade lasers as described above, is arranged in a second segment 202B of the holding portion 104 that is different from the first segment 202A, in which the measurement body 106 (as well as the controller and display) are arranged. The second segment 202B may e.g. be a segment adjacent to the first segment 202. This may for example be advantageous since the excitation radiation source 26 may generate a sizeable amount of heat when generating the excitation radiation, which may affect temperature-sensitive components such as an OLED display or the controller, but also the analyte measurement, e.g. by changing a temperature of the measurement body 16. The second segment 202B may be configured to form a heat sink for the excitation radiation source 26. The second segment 202B may be larger than the first segment 202A in at least one physical dimension, for example in the circumferential extent around the body part as illustrated in Fig. 10.

An output of the excitation radiation source 26 is coupled to the measurement body 16 via a waveguide 302, e.g. a multimode optical fiber, such that the excitation radiation source 26 may irradiate excitation radiation into the tissue through the measurement body 16, e.g. as shown in Fig. 1. The waveguide 302 may for example be arranged in an interior of the segments 202A, 202B and may extend through or adjacent to the hinge 204 between the segments 202A, 202B.

The wearable device 300 further comprises a spacer 304 for thermally insulating the body part (not shown) that the wearable device 300 is secured to from the excitation radiation source 26. The spacer 304 is arranged between the excitation radiation source 26 and an inner surface of the wearable device 300 that faces the body part 106 when the wearable device is secured to the body part. In the example of Fig. 10, the spacer 304 is arranged on an inner surface of the second segment 202B such that an inner surface of the spacer 304 forms part of the inner surface of the wearable device 300. In other examples, the spacer 304 may for example form a part of an inner sidewall of the segment 202B, i.e. the sidewall of the segment 202B facing the body part, or may be arranged inside the segment 202B on the inner sidewall of the segment 202B. The spacer 304 may have a lower thermal conductivity than sidewalls of the segment 202B and/or of the first segment 202A, e.g. a thermal conductivity that is at least a factor of five, preferably at least a factor of ten smaller than the thermal conductivity of sidewalls of the second segment 202B and/or of the first segment 202A. Additionally or alternatively, the spacer 304 may be configured to maintain at least a minimum distance between the skin of the subject and the excitation radiation source 26 and/or the inner sidewall of the second segment 202B. The spacer 304 may for example comprise a plurality of ridges or fins that are configured to prevent the inner sidewall of the second segment 202B from coming in contact with the skin, e.g. to keep the inner sidewall of the second segment 202B at least the minimum distance away from the skin.

The wearable device 300 also comprises a removable battery 306 that is configured to be removably coupled to the excitation radiation source 26, e.g. for supplying energy thereto while the excitation radiation is being generated. The wearable device 300 may further comprise an integrated battery (not shown), which may e.g. be permanently mounted within the main housing 102 and configured to supply energy to at least the controller, the display and the detection device (not shown). The excitation radiation source 26, however, may require more energy than the integrated battery can provide and/or may quickly drain the integrated battery when generating the excitation radiation.

The removable battery 306 may be configured to supply energy to the excitation radiation source 26 through a wired electrical connection, e.g. via a connector and/or contact pin or pads arranged on the second segment 202B, and/or through a wireless electrical connection, e.g. an inductive coupling. In some examples, the removable battery 306 may additionally or alternatively be configured to supply energy to the integrated battery, e.g. to recharge the integrated battery prior to or after performing an analyte measurement. The removable battery 306 may be configured to be removably attached to the holding portion 104, in particular to the second segment 202B. The removable battery 306 and/or the holding portion 104 may for example comprise mechanical fasteners such as clips or hooks and/or one or more magnets for removably attaching the removable battery 306 to the holding portion 104.

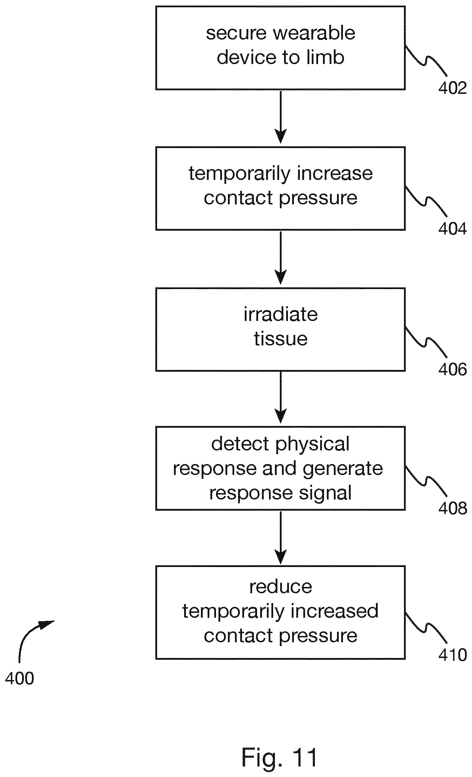

Fig. 11 shows a flow chart of a method 400 for detecting an analyte in tissue of a human or animal subject according to an exemplary embodiment of the invention. The method 400 may be executed with a wearable device for detecting an analyte in tissue of a human or animal subject according to any one of the embodiments described herein. In the following, the method 400 is described using the wearable device 100 as a non-limiting example for illustration purposes. This is, however, not intended to be limiting in any way and the method 400 may also be executed using a different wearable device, for example any one of the wearable devices 200, 200' and 300. Furthermore, the method 400 is not limited to the order of execution implied by the flow chart in Fig. 11. As far as technically feasible, the method 400 may be executed in an arbitrary order and parts thereof may be executed simultaneously at least in part, e.g. steps 406 and 408.

In some examples, the method 400 may be executed by the controller 34 of the wearable device 100 at least in part (e.g. steps 404 to 410). The controller 34 may for example be configured to control the excitation radiation source, the detection device and the actuated means 110 for temporarily increasing a contact pressure between the measurement body 16 and the skin of the subject to perform the respective actions. In other words, steps 404 to 410 may be executed automatically without human input or interference.

The method 400 comprises, in step 402, securing the wearable device 100 to a body part 106 of the subject, for example to a wrist of the subject similar to a wristwatch. The wearable device 100 is secured such that the contact surface 14 of the measurement body 16 faces a skin of the subject, preferably such that the contact surface 14 is in contact with the skin. The holding portion 104 may for example comprise a buckle or clasp that can be opened and closed for securing the wearable device 100 to the wrist of the subject.

The method 400 further comprises, in step 404, temporarily increasing a contact pressure between the measurement body 16 and the skin of the subject. The contact pressure may for example be increased in response to determining that an analyte measurement is to be performed. The analyte measurement may for example be performed following a corresponding user command and/or automatically, e.g. on a regular basis with a pre-defined time delay between subsequent measurements.

The contact pressure may for example be increased by moving the measurement body 16 radially inward towards the body part 106 with the actuator 110 as illustrated in Figs. 7a, 7b. This may comprise determining the contact pressure, e.g. by performing a measurement with the excitation radiation source 26 and the detection device 28, 30 and/or using an auxiliary sensor such as the pulse and/or oxygen saturation sensor 210. In some examples, the contact pressure may be increased by moving the measurement body 16 radially inward until the contact pressure meets or exceeds a pre-defined threshold.

Additionally or alternatively, the contact pressure may also be increased by reducing one or both of an inner diameter d and an inner circumference C of the wearable device 100. Step 404 may for example comprise inflating and/or deflating an inflatable body in the holding portion 104, e.g. inflating one or more inflatable segments 208 as illustrated in Fig. 9a. Step 404 may also comprise actuating an actuated hinge such as the hinge 206 in Fig. 9a.

In steps 406 and 408, one or more analyte measurements are performed while maintaining the temporarily increased contact pressure. For this, excitation radiation is irradiated into the tissue to be absorbed therein using the excitation radiation source 26 in step 406, e.g. at a plurality of analyte-characteristic wavelengths as described above. Using the detection device 28, 30, the physical response of the measurement body 16 or a component included therein is detected and a response signal indicative of the degree of absorption of said excitation radiation is generated in step 408. In some examples, the method 400 may also comprise determining a measure for the concentration of the analyte in the tissue based on the response signal.

In some embodiments, the method 400 may also comprise determining the contact pressure between the measurement body 16 and the skin of the subject prior to, during and/or after performing the analyte measurement, e.g. as described above. If the contact pressure is below a pre-defined threshold, the analyte measurement may be discarded and/or the contact pressure may be increased further, e.g. to repeat the analyte measurement.

After performing the analyte measurement, the temporarily increased contact pressure is reduced again in step 410. Preferably, the temporarily increased contact pressure is released completely, e.g. to return the wearable device 100 to the state prior to increasing the contact pressure in step 404. Accordingly, step 410 may comprise reversing some or all of actions taking in step 404 for increasing the contact pressure, e.g. by moving the measurement body 16 back to its initial position using the actuator 110 and/or by deflating the inflatable segments 208.

Fig. 12 shows a schematic illustration of a wearable device 400 for detecting an analyte in tissue of a human or animal subject according to an exemplary embodiment of the invention. The wearable device 400 is similar to the wearable device 100 of Fig. 1 and also comprises a main housing 102, a holding portion 104, a display 108 and an apparatus for detecting the analyte (e.g. glucose) in tissue of a body part 106, wherein the apparatus comprises a measurement body 16 having a contact surface 14.

Instead of (or in other embodiments in addition to) the actuated means for temporarily increasing a contact pressure between the measurement body 16 and the skin of the subject, the wearable device 400 comprises a contact monitor 402 for monitoring a thermal contact state and/or a pressure transmitting contact state between the contact surface 14 of the measurement body 16 and the skin of the subject. The contact monitor 402 may for example comprise a contact sensor (not shown) such as a pressure sensor, an electrical sensor (e.g. a capacitive sensor, an inductive sensor, a voltmeter and/or an ohmmeter) and/or an optical sensor (e.g. an optical sensor configured to measure an intensity of light reflected from the body part and/or an intensity of light reflected at an interface between the wearable device 400 and the body part 106, e.g. at an inner surface of the wearable device such as the contact surface 14). A sensor signal generated by the contact sensor and/or a parameter measured by the contact sensor may depend on the contact state between the contact surface 14 of measurement body 16 and the skin of the subject (e.g. on the contact pressure and/or on whether or not there is a contact between the contact surface 14 and the skin). The contact monitor 402 may be configured to determine whether there is a contact between the skin of the subject and the contact surface 14 of the measurement body 16 using the contact sensor, e.g. by determining whether a pressure force measured by the pressure sensor exceeds a pre-defined threshold and/or by determining whether an electric resistance measured by the electrical sensor is below a pre-defined threshold.

The contact monitor 402 may be configured to initiate and/or prevent execution of an analyte measurement depending on whether there is contact between the skin of the subject and the contact surface 14 of the measurement body 16, e.g. by sending a corresponding control signal to the controller (not shown) of the wearable device 400.

While the present invention has been described in terms of specific embodiments, it is understood that variations and modifications will occur to those in the art, all of which are intended as aspects of the present invention. Accordingly, only such limitations as appear in the claims should be placed on the invention.

**Claims**

1. A wearable device (100, 200, 300) for detecting an analyte in tissue of a human or animal subject, the wearable device (100, 200, 300) comprising:

   a measurement body (16) having a contact surface (14) suitable to be brought in contact with a skin of the subject, said contact permitting heat and/or pressure waves generated by absorption of excitation radiation in the tissue to be transferred to said measurement body (16);
   an excitation radiation source (26) configured for irradiating excitation radiation at a plurality of wavelengths into the tissue to be absorbed by the analyte contained therein;
   a detection device (28, 30) for detecting a physical response of the measurement body (16) or of a component included therein to a heat and/or pressure wave received from the tissue upon absorption of the excitation radiation and for generating a response signal based on said detected physical response, said response signal being indicative of a degree of absorption of excitation radiation;
   a controller (34) to control the excitation radiation source (26) to irradiate excitation radiation into the tissue to be absorbed by the analyte contained therein and to control the detection device (28, 30) to detect said physical response and
   to generate a response signal indicative of the degree of absorption of said excitation radiation; and
   a holding portion (104) for securing the wearable device (100, 200, 300) to a body part (106), in particular a limb of the subject, said holding portion (104) being configured to be arranged around the body part (106) of the subject so as to extend circumferentially around the body part (106) at least in part;

   wherein the wearable device (100, 200, 300) further comprises actuated means (110, 206, 208) for temporarily increasing a contact pressure between the measurement body (16) and the skin of the subject by reversibly performing one or more of the following:

   reducing an inner diameter (d) of the wearable device (100, 200, 300), wherein the inner diameter (d) of the wearable device (100, 200, 300) is a distance between two opposing portions of the wearable device (100, 200, 300) being arranged on opposite sides of the body part (106) when the wearable device (100, 200, 300) is secured to the body part (106);
   reducing an inner circumference (C) of the wearable device (100, 200, 300), wherein the inner circumference (C) of the wearable device (100, 200, 300) is a length of an inner surface of the wearable device (100, 200, 300) facing the body part (106) when the wearable device (100, 200, 300) is secured to the body part (106); and moving the measurement body (16) or a part thereof radially inward towards the body part (106).

2. The wearable device (100, 200, 300) of claim 1, wherein said analyte is glucose, in particular wherein said tissue is the skin of the subject and said analyte is glucose present in an intersitital fluid thereof.

3. The wearable device (100, 200, 300) of claim 1 or 2, wherein the actuated means (110, 206, 208) for temporarily increasing the contact pressure comprise one or more actuators (110), the one or more actuators including one or more of an electric actuator, a magnetic actuator, a piezoelement and a fluid pump, and/or
wherein the actuated means (110, 206, 208) for temporarily increasing the contact pressure comprise one or more deformable members (208) configured to be deformed, extended and/or retracted reversibly, the one or more deformable members comprising one or more of a bimetallic element, a shape-memory alloy structure, a bistable spring assembly, a pneumatically and/or hydraulically extendable member and an inflatable body.

4. The wearable device (100, 200, 300) of claim 3, wherein at least one of the one or more actuators (110) and/or at least one of the one or more deformable members (208) is arranged on a same side or an opposite side of the body part (106) as the measurement body (16) when the wearable device (100, 200, 300) is secured to the body part (106), and/or wherein the measurement body (16) is mounted movably on and/or in the wearable device (100, 200, 300) and:
the one or more actuators (110) comprise an actuator configured to move the measurement body (16) radially inward towards the body part (106); and/or the one or more deformable members (208) comprise a deformable member configured to move the measurement body (16) radially inward towards the body part (106).

5. The wearable device (100, 200, 300) of any one of the preceding claims, wherein the holding portion (104) comprises a plurality of segments (202) connected with each other to form a chain,

wherein the actuated means (110, 206, 208) for temporarily increasing the contact pressure are preferably configured to reversibly reduce a circumferential extent of one or more segments (202) of the holding portion (104) and/or a distance between one or more pairs of adjacent segments (202) of the holding portion (104), and/or
wherein the actuated means (110, 206, 208) for temporarily increasing the contact pressure comprise at least one actuated hinge (206) connecting a pair of adjacent segments (202) of the holding portion (104), wherein the actuated hinge (206) is configured to rotate the pair of adjacent segments (202) with respect to each other to move the actuated hinge (206) radially inward towards the body part (106).

6. The wearable device (100, 200, 300) of claim 5, wherein the plurality of segments (202) comprises a first segment (202A) in and/or on which the controller is arranged and a second segment (202B) in and/or on which the excitation radiation source (26) is arranged, the second segment (202B) being different from the first segment (202A),

wherein the first segment (202A) is preferably a main housing (102) of the wearable device (100, 200, 300) and the main housing (102) further comprises a display (108) configured to display a measurement result indicative of the degree of absorption of the excitation radiation and/or of a presence and/or a concentration of the analyte in the tissue,
wherein the second segment (202) is preferably larger than the first segment (202A) in at least one physical dimension, in particular wherein a circumferential extent and/or a volume of the second segment (202B) is larger than a circumferential extent and a volume, respectively, of the first segment (202A).

7. The wearable device (100, 200, 300) of any one of the preceding claims, wherein the measurement body (16) and the excitation radiation source (26) are arranged in different parts of the wearable device (100, 200, 300), in particular in different segments (202) of the holding portion (104), and the wearable device (100, 200, 300) further comprises a waveguide (302) configured to guide excitation radiation from the part of the wearable device (100, 200, 300) in which the excitation radiation source (26) is arranged to the part of the wearable device (100, 200, 300) in which the measurement body (16) is arranged.

8. The wearable device (100, 200, 300) of any one of the preceding claims, further comprising a spacer (304) for thermally insulating the body part (106) from the excitation radiation source (26), the spacer (304) being arranged between the excitation radiation source (26) and an inner surface of the wearable device (100, 200, 300) facing the body part (106) when the wearable device (100, 200, 300) is secured to the body part (106), and/or wherein the measurement body (16) or a part thereof permanently protrudes from the inner surface of the wearable device (100, 200, 300) and/or wherein the measurement body (16) is arranged in and/or on a permanent protrusion on the inner surface of the wearable device (100, 200, 300).

9. The wearable device (100, 200, 300) of any one of the preceding claims, wherein the wearable device (100, 200, 300) comprises a removable battery (306) configured to be removably coupled to an integrated battery of the wearable device (100, 200, 300) and/or to the excitation radiation source (26) for supplying energy to the integrated battery and/or to the excitation radiation source (26), wherein the wearable device (100, 200, 300) preferably comprises an integrated battery configured to supply energy to the detection device (28, 30) and/or to the controller (34), wherein the removable battery (306) is configured to be removably attached to the wearable device (100, 200, 300) for supplying energy to the excitation radiation source (26) while irradiating excitation radiation into the tissue.

10. The wearable device (100, 200, 300) of any one of the preceding claims, further comprising an auxiliary sensor (210) configured to generate a sensor signal that depends on the contact pressure between the measurement body (16) and the skin of the subject, wherein the auxiliary sensor (210) is preferably configured to measure one or more of a blood pressure of the subject, a pulse of the subject and an oxygen saturation of blood of the subject.

11. The wearable device (100, 200, 300) of any one of the preceding claims, wherein the controller (34) is configured to determine the contact pressure between the measurement body (16) and the skin of the subject based on a response signal from the detection device (28, 30) and/or based on a sensor signal from the auxiliary sensor (210), wherein the controller (34) is preferably configured to determine the contact pressure by:

controlling the excitation radiation source (26) to irradiate excitation radiation into the tissue to be absorbed therein at one or more wavelengths at which the degree of absorption of said excitation radiation is substantially independent of a concentration of the analyte in the tissue; and controlling the detection device (28, 30) to detect said physical response and to generate a response signal indicative of the degree of absorption of said excitation radiation, and/or

wherein the controller (34) is preferably configured to discard a response signal generated by the detection device (28, 30) for detection of the analyte if the determined contact pressure is below a threshold, and/or wherein the wearable device (100, 200, 300) further comprises a contact monitor (402) for monitoring a thermal contact state and/or a pressure transmitting contact state between the contact surface (14) of the measurement body (16) and the skin of the subject.

12. A method (400) for detecting an analyte in tissue of a human or animal subject using a wearable device (100, 200, 300) according to any one of the preceding claims, the method (400) comprising:

securing the wearable device (100, 200, 300) to a body part (106), in particular a limb of the subject such that the contact surface (14) of the measurement body (16) faces a skin of the subject;
temporarily increasing a contact pressure between the measurement body (16) and the skin of the subject by one or more of reducing the inner diameter (d) of the wearable device (100, 200, 300), reducing the inner circumference (C) of the wearable device (100, 200, 300) and moving the measurement body (16) or a part thereof radially inward towards the body part (106);
while maintaining the temporarily increased contact pressure, irradiating excitation radiation into the tissue to be absorbed therein using the excitation radiation source (26), detecting the physical response of the measurement body (16) or a component included therein and generating a response signal indicative of the degree of absorption of said excitation radiation using the detection device; and
reducing the temporarily increased contact pressure while the wearable device (100, 200, 300) remains secured to the body part (106) of the subject, wherein

the method (400) preferably further comprises determining the contact pressure between the measurement body (16) and the skin of the subject, wherein the response signal is discarded for detection of the analyte if the determined contact pressure is below a threshold.

13. A wearable device (400) for detecting an analyte in tissue of a human or animal subject, the wearable device (400) comprising:

a measurement body (16) having a contact surface (14) suitable to be brought in contact with a skin of the subject, said contact permitting heat and/or pressure waves generated by absorption of excitation radiation in the tissue to be transferred to said measurement body (16);

an excitation radiation source (26) configured for irradiating excitation radiation at a plurality of wavelengths into the tissue to be absorbed by the analyte contained therein;

a detection device (28, 30) for detecting a physical response of the measurement body (16) or of a component included therein to a heat and/or pressure wave received from the tissue upon absorption of the excitation radiation and for generating a response signal based on said detected physical response, said response signal being indicative of a degree of absorption of excitation radiation;

a controller (34) to control the excitation radiation source (26) to irradiate excitation radiation into the tissue to be absorbed by the analyte contained therein and to control the detection device (28, 30) to detect said physical response and to generate a response signal indicative of the degree of absorption of said excitation radiation; and

a holding portion (104) for securing the wearable device (400) to a body part (106), in particular a limb of the subject, said holding portion (104) being configured to be arranged around the body part (106) of the subject so as to extend circumferentially around the body part (106) at least in part;

wherein the wearable device (400) further comprises a contact monitor (402) for monitoring a thermal contact state and/or a pressure transmitting contact state between the contact surface (14) of the measurement body (16) and the skin of the subject.

14. The wearable device (400) according to claim 13, wherein the contact monitor (402) comprises one or more contact sensors, said one or more contact sensors comprising one or more of:

a pressure sensor configured to measure a pressure force between the measurement body (16) and the skin of the subject;

an electrical sensor configured to measure an electric resistance and/or an electric capacitance and/or an electric voltage between the measurement body (16) and the skin of the subject and/or an inductance of an inductive element of the wearable device;

a humidity sensor configured to measure a humidity between the measurement body (16) and the skin of the subject;

a fingerprint sensor configured to recognize a fingerprint on the contact surface (14) of the measurement body (16); and

an optical sensor configured to measure a reflection of light at the contact surface (14) of the measurement body (16),

wherein said contact monitor (402) is configured to determine whether there is a contact between the skin of the subject and the contact surface (14) of the measurement body (16) using said one or more contact sensors.

15. The wearable device (400) according to claim 13 or 14, wherein the contact monitor (402) comprises a measurement module which is configured to detect, upon an activity of the excitation radiation source (26) irradiating excitation radiation into the tissue, whether there is a physical response of the measurement body (16) or of a component included therein, in particular by using the detection device (28, 30), and/or

wherein the contact monitor (402) is configured to generate an alarm and/or to block the execution of an analyte measurement based on said thermal contact state and/or said pressure transmitting contact state, and/or

wherein the wearable device (400) further comprises actuated means (110, 206, 208) for temporarily increasing a contact pressure between the measurement body (16) and the skin of the subject and the contact monitor (402) is configured to activate the actuated means (110, 206, 208) for temporarily increasing a contact pressure between the measurement body (16) and the skin of the subject based on said thermal contact state and/or said pressure transmitting contact state.

**Fig. 1**

IR spectra of glucose at different concentrations

**Fig. 2**

**Fig. 3**

Clarke's Error Grid Analysis-raw Amplitude

1558 data points

20 subjects
of these
17 healthy,
2 type 1
diabetics,
1 type 2
diabetic

zone A: 95.4%
zone B: 4.0%
zone C: 0%
zone D: 0.6%
zone E: 0%

**Fig. 4**

**Fig. 5**

**Fig. 6**

Fig. 7a

Fig. 7b

102
108
102A
102B
106
16

Fig. 8a

102
108
102A
110
110
102B
16
106

Fig. 8b

200
210
102
104
16
202
204
208
208
d
C
206

Fig. 9a

16
202
200'
206

Fig. 9b

Fig. 10

```
┌─────────────────────┐
│   secure wearable   │
│   device to limb    │╮
└─────────────────────┘│
          │         402
          ▼
┌─────────────────────┐
│ temporarily increase│
│  contact pressure   │╮
└─────────────────────┘│
          │         404
          ▼
┌─────────────────────┐
│     irradiate       │
│      tissue         │╮
└─────────────────────┘│
          │         406
          ▼
┌─────────────────────┐
│  detect physical    │
│ response and generate│
│  response signal    │╮
└─────────────────────┘│
          │         408
          ▼
┌─────────────────────┐
│      reduce         │
│ temporarily increased│
│  contact pressure   │╮
└─────────────────────┘│
                    410
```

400

Fig. 11

Fig. 12

Europäisches
Patentamt

European
Patent Office

Office européen
des brevets

## EUROPEAN SEARCH REPORT

Application Number

EP 22 15 8049

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2018/328835 A1 (BAUER ALEXANDER [DE] ET AL) 15 November 2018 (2018-11-15)<br>* figures 1, 8, 9, 23 *<br>* paragraphs [0098] – [0099], [0119] – [0123], [0131] – [0133], [0153] – [0154], [0157] *<br>* paragraphs [0163], [0171], [0181] – [0183] * | 1-7,<br>10-12<br>8,9 | INV.<br>A61B5/145<br>A61B5/00 |
| X | WO 2021/239263 A1 (DIAMONTECH AG [DE]) 2 December 2021 (2021-12-02)<br>* figure 17 *<br>* page 12, lines 5-36 * | 1 | |
| Y | US 2021/401291 A1 (SCHRIEK UWE [DE] ET AL) 30 December 2021 (2021-12-30)<br>* paragraphs [0077], [0224] *<br>* figure 23 * | 8 | |
| Y | US 2006/200017 A1 (MONFRE STEPHEN L [US] ET AL) 7 September 2006 (2006-09-07)<br>* paragraph [0107] * | 9 | TECHNICAL FIELDS SEARCHED (IPC)<br><br>A61B |
| A | US 2015/112170 A1 (AMERSON III ROBERT LEE [US] ET AL) 23 April 2015 (2015-04-23)<br>* paragraphs [0051], [0052] *<br>* figure 5 * | 5-7 | |

The present search report has been drawn up for all claims

| Place of search | Date of completion of the search | Examiner |
|---|---|---|
| Munich | 8 July 2022 | Trattner, Barbara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................
& : member of the same patent family, corresponding
   document

EPO FORM 1503 03.82 (P04C01)

Europäisches
Patentamt

European
Patent Office

Office européen
des brevets

**Application Number**

**EP 22 15 8049**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-12**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

**Application Number**

**EP 22 15 8049**

The Search Division considers that the present European patent application does not comply with the requirements of unity of invention and relates to several inventions or groups of inventions, namely:

    **1. claims: 1-12**

        **Device and method for detecting an analyte in tissue comprising actuators to adjust the contact pressure between the device and the body**
        ---

    **2. claims: 13-15**

        **Device for detecting an analyte in tissue comprising a contact monitor to determine the contact state between the device and the body**
        ---

## ANNEX TO THE EUROPEAN SEARCH REPORT
## ON EUROPEAN PATENT APPLICATION NO.

EP 22 15 8049

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2022

| Patent document cited in search report | | Publication date | Patent family member(s) | | Publication date |
|---|---|---|---|---|---|
| US 2018328835 | A1 | 15-11-2018 | CN 108369183 | A | 03-08-2018 |
| | | | EP 3359949 | A1 | 15-08-2018 |
| | | | EP 3524962 | A1 | 14-08-2019 |
| | | | JP 2019507319 | A | 14-03-2019 |
| | | | KR 20180091874 | A | 16-08-2018 |
| | | | US 2018328835 | A1 | 15-11-2018 |
| | | | US 2018335381 | A1 | 22-11-2018 |
| | | | US 2022205909 | A1 | 30-06-2022 |
| | | | WO 2017097824 | A1 | 15-06-2017 |
| WO 2021239263 | A1 | 02-12-2021 | NONE | | |
| US 2021401291 | A1 | 30-12-2021 | BR 112021007850 | A2 | 03-08-2021 |
| | | | CA 3117153 | A1 | 14-05-2020 |
| | | | CN 112955075 | A | 11-06-2021 |
| | | | EP 3876840 | A1 | 15-09-2021 |
| | | | JP 2022506275 | A | 17-01-2022 |
| | | | KR 20210089687 | A | 16-07-2021 |
| | | | US 2021401291 | A1 | 30-12-2021 |
| | | | WO 2020094233 | A1 | 14-05-2020 |
| | | | WO 2020094265 | A1 | 14-05-2020 |
| US 2006200017 | A1 | 07-09-2006 | EP 1850736 | A2 | 07-11-2007 |
| | | | JP 2008531133 | A | 14-08-2008 |
| | | | US 2006200017 | A1 | 07-09-2006 |
| | | | WO 2006093816 | A2 | 08-09-2006 |
| US 2015112170 | A1 | 23-04-2015 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015193310 A1
- WO 2017097824 A1
- EP 2019064356 W
- WO 2019110597 A2
- WO 2021233559 A1
- WO 201709782 A1
- WO 201911059782 A